# EUROPEAN PATENT APPLICATION

(11) **EP 4 671 376 A1**
(43) Date of publication of application: **31.12.2025**
(21) Application number: 24760261.8
(22) Date of filing: 16.02.2024
(51) Int. Cl.: C12P 21/02, A61K 38/38, A61K 47/61, A61P 35/00, C07K 1/13, C07K 14/47

(54) **PROTEIN RECOGNIZING K-RAS GENE MUTATION CANCER**

(30) Priority: 20.02.2023 JP 2023024543
(71) Applicant: RIKEN, Wako-shi, Saitama 351-0198 (JP); Glytech, Inc., Kyoto-shi, Kyoto 600-8813 (JP)
(72) Inventor: TANAKA, Katsunori, Wako-shi, Saitama 351-0198 (JP); CHANG, Tsung-che, Wako-shi, Saitama 351-0198 (JP); SHIMODA, Taiji, Kyoto-shi, Kyoto 600-8813 (JP)
(74) Representative: Greaves Brewster LLP
(86) International application number: PCT/JP2024/005473
(87) International publication number: WO 2024/176961

(57) **Abstract**

The object of the present disclosure is to provide a protein that selectively recognizes cancer caused by a mutation of K-Ras gene (herein particularly referred to as "K-Ras gene mutation cancer"). Provided are a production method of a protein that selectively recognizes K-ras gene mutation cancer characterized in that it comprises first to third steps of linking first to third specific sugar chains respectively to lysine residues on the protein molecule, as well as the protein produced by the aforementioned method. Moreover, a pharmaceutical composition for cancer therapy comprising said protein is provided.

## Description

### Technical Field

The present disclosure relates to a protein that recognizes K-Ras gene mutation cancer, as well as a production method thereof.

### Background Art

Currently, attempts related to *"in vivo* synthetic chemotherapy" that utilizes catalysts are under investigation. *In vivo* synthetic chemotherapy is an attempt to introduce a source material or reagent without activity or toxicity into the body, and activating the aforementioned source material or reagent at a particular location inside the body by a catalyst to exert effect. There is an increasing interest towards development of novel catalysts that are applicable to therapeutic application (Non-Patent Literature 1).

One problem in the development of such catalysts is the construction of a drug delivery system (DDS) that selectively targets a particular target. So far, there have been attempts to generate molecules that selectively recognize particular targets (such as cancer cells) by modifying the surface of a catalytic molecule (typically a protein catalytic molecule) with various sugar chains (Patent Literature 1; Non-Patent Literature 2).

### Citation List

### Patent Literatures

[Patent Literature 1] International Publication No. 2020/241340 Non-Patent Literatures

[Non-Patent Literature 1] Rebelein, J.G. ; Ward, T. R. Curr. Opin. Biotechnol. 2018, 53, 106-114.
[Non-Patent Literature 2] Katsumasa Fujiki and Katsunori Tanaka, "RIKEN Click Reagent for Protein Labeling", Encyclopedia of Reagents for Organic Synthesis (e-EROS)

### Summary of the Invention

### Problems to be Solved by the Invention

K-Ras gene is a representative gene that is associated with oncogenesis of many cancers, and therapeutic drugs effective for cancer caused by K-Ras gene mutation is limited to only a small fraction.

The object of the present disclosure is to provide a protein that selectively recognizes cancer caused by a mutation of K-Ras gene (herein particularly referred to as "K-Ras gene mutation cancer").

### Means for Solving the Problems

As a result of extensive investigation in order to achieve the above objectives, it has been found that selectivity for K-Ras gene mutation cancer can be rendered to the aforementioned protein by conjugating sugar chains that have particular structures onto the protein surface in a particular order.

In other words, the present disclosure non-limitingly encompasses the following characteristics.
(1) A production method of a protein that selectively recognizes K-ras gene mutation cancer, comprising:
   a first step of conjugating the following sugar chain to lysine residues on the protein molecule; 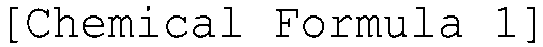 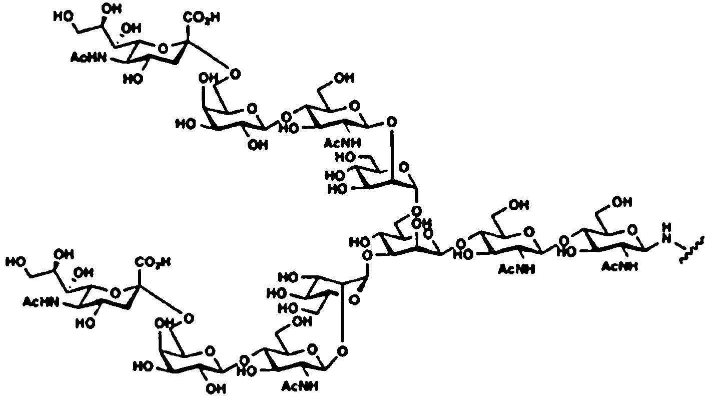
   a second step of conjugating the following sugar chain to lysine residues on said molecule; and 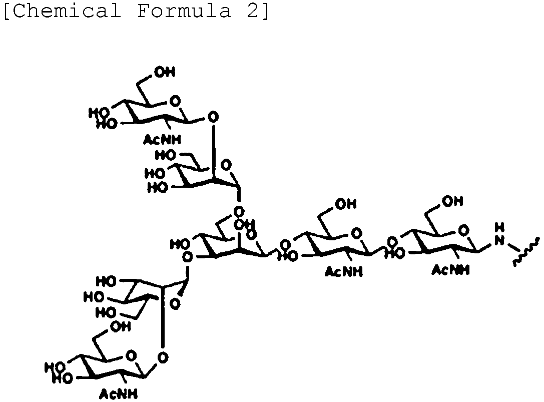
   a third step of conjugating any of multiantennary complex sugar chains to lysine residues on said molecule.
(2) The production method according to (1), wherein said multiantennary complex sugar chain is at least one type of biantennary complex sugar chain.
(3) The production method according to (2), wherein in the third step, at least one of sugar chain selected from the group consisting of the following is conjugated: 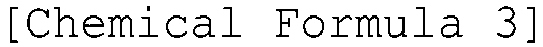 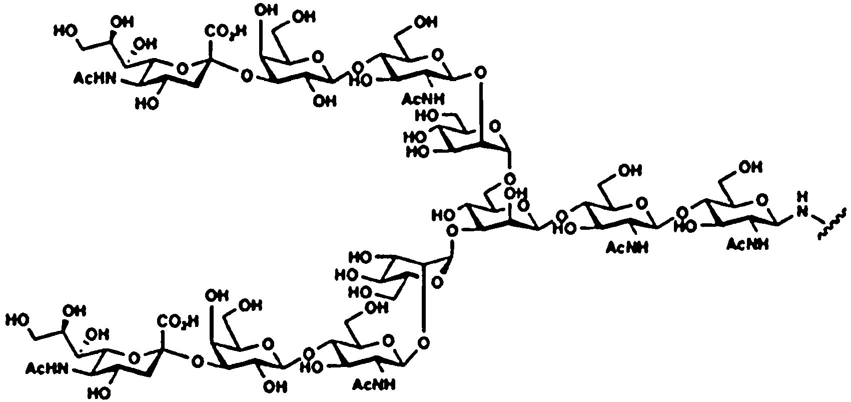 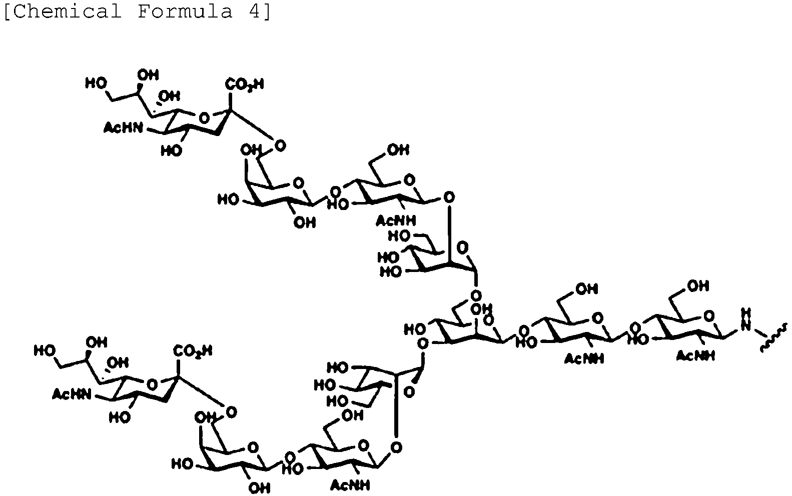 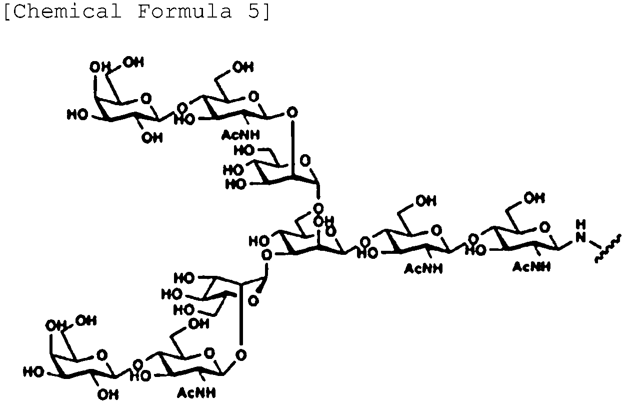 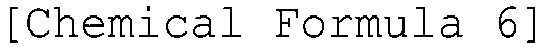 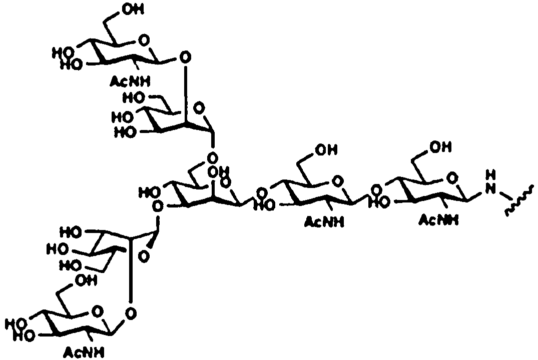 and 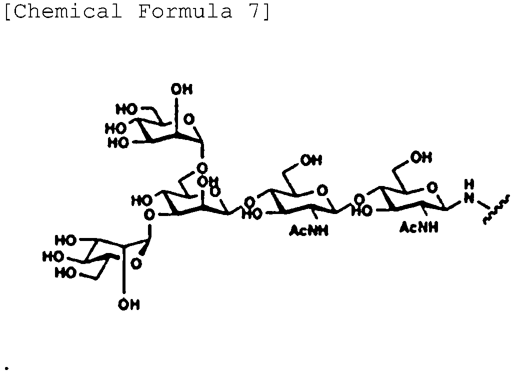
(4) The production method according to claim 2, wherein in the third step, at least any one of the following sugar chains is conjugated: 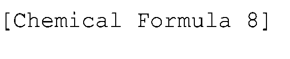 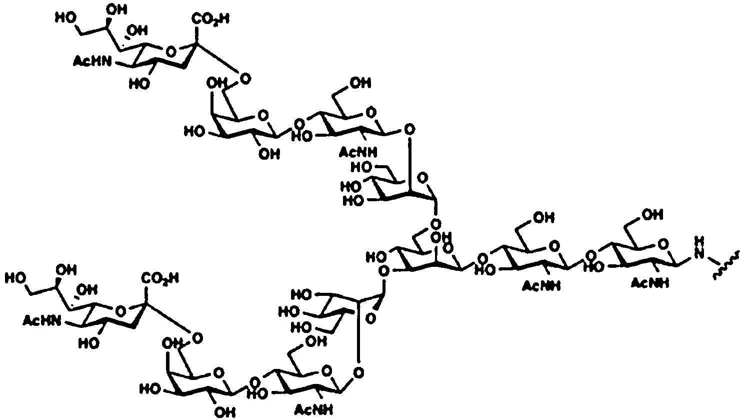 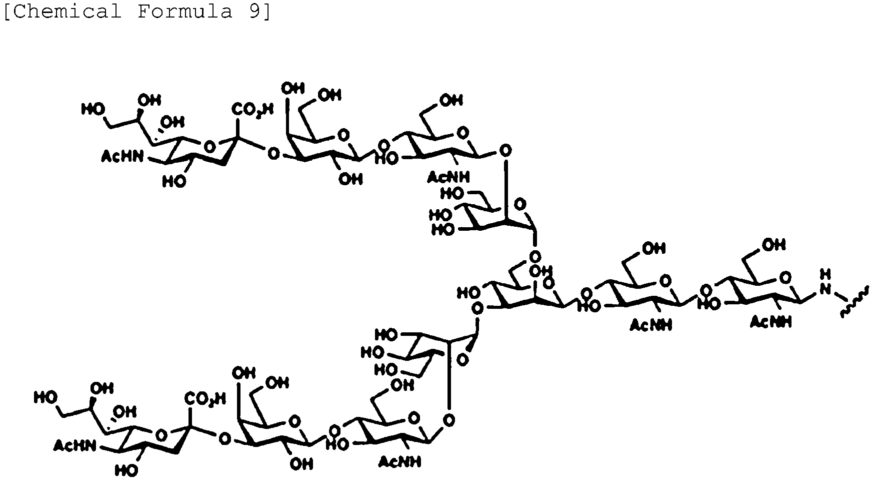 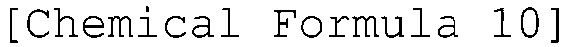 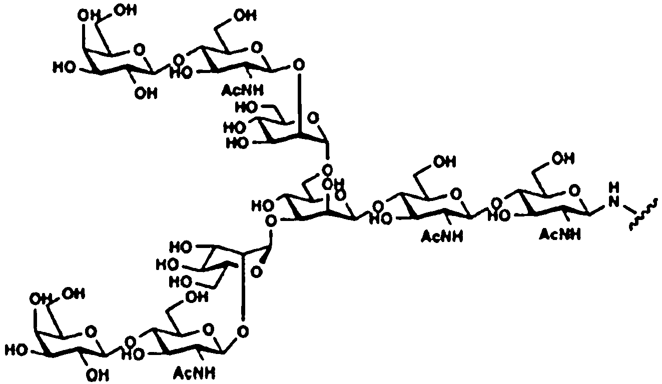 ; and 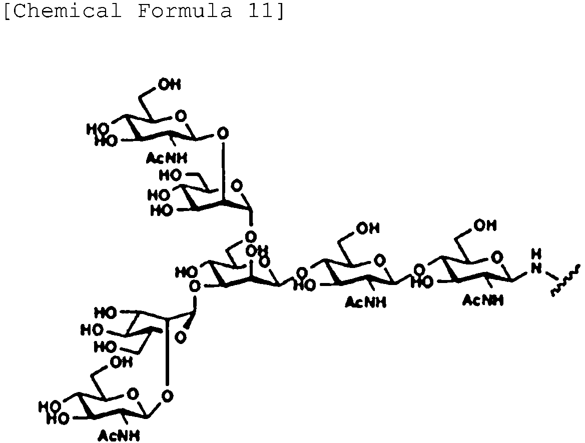 .
(5) The production method according to (1), wherein said protein is albumin.
(6) The method according to (1), wherein in each of said first to third steps, 2 to 5 sugar chains are respectively conjugated.
(7) The method according to (1), wherein in each of said first to third steps, about 3 sugar chains are respectively conjugated.
(8) The production method according to (1), wherein said "protein that selectively recognizes K-ras gene mutation cancer" comprises 9 to 11 sugar chains.
(9) The production method according to (1), wherein said "K-ras gene mutation" comprises a G12D mutation.
(10) The production method according to (1), wherein said "K-ras gene mutation cancer" is gastric adenocarcinoma or pancreatic cancer.
(11) The production method according to (5), wherein said albumin is a protein catalyst that accommodates a metal catalyst in its hydrophobic spot so that it is not exposed or not substantially exposed to the hydrophilic environment.
(12) The production method according to (11), wherein said hydrophobic spot is albumin drug binding site I (drug site I).
(13) The production method according to (11), wherein said metal catalyst is bound to said hydrophobic spot via a ligand against said albumin.
(14) The production method according to (13), wherein said ligand is selected from the group consisting of warfarin, azapropazone, acenocoumarol, phenylbutazone, salicylate salt, indomethacin, phenytoin, tolbutamide, chlorpropamide, iophenoxate, iodipamide, sulfadimethoxine, phenprocoumon, glibenclamide, sulfathiazole, tenoxicam, camptothecin, prodan, bilirubin, eicosanoid, and carboxy-methyl-propyl-furan propanoate (uremic toxin), and coumarin.
(15) The production method according to (13), wherein said metal catalyst is bound to said hydrophobic spot via a linker bound to said ligand.
(16) The production method according to (15), wherein said linker is an alkyl chain or a polyethylene glycol (PEG) chain having amino and carboxyl groups on both ends.
(17) The production method according to (16), wherein said linker is C₁ - C₃ alkyl.
(18) The production method according to (11), wherein said metal catalyst is a ruthenium catalyst.
(19) A protein that selectively recognizes K-ras gene mutation cancer that can be produced by the production method according to any of (1) to (18).
(20) A pharmaceutical composition for cancer therapy comprising the protein according to (19).

Those skilled in the art will recognize that a configuration of any combination of one or more characteristics of the present disclosure described above is also within the scope of the present disclosure.

### Effects of the Invention

According to the present disclosure, a protein molecule that selectively recognizes cancer caused by a mutation of K-Ras gene is provided.

### Brief Description of the Drawings

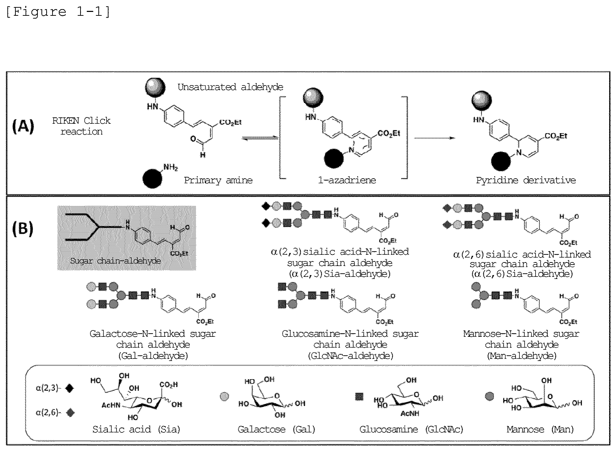

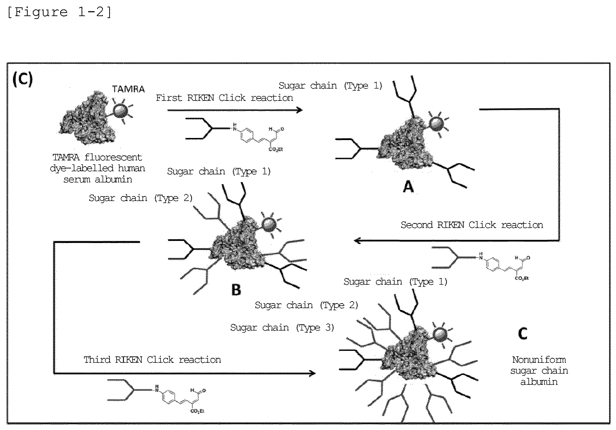

Figure 1 shows an overview of the synthetic means that can be employed in the present disclosure. (A) shows application of RIKEN Click reaction for the modification of protein lysine residues via 6π-aza electron cyclization reaction of unsaturated imine. (B) shows the structures and symbols of the N-linked sugar chain-aldehyde compounds that can be used in the present disclosure. (C) illustrates the means for synthesizing a sugar chain albumin having three different types of N-linked sugar chains by sequentially conjugating sugar chain aldehydes to human serum albumin bound to TAMRA fluorescent substance.
Figure 2 shows the entire chemical structures of the sugar chain aldehyde compounds used in the present Example.
Figure 3 shows the molecular weights of albumin A1 - A5.
Figure 4 shows the molecular weights of sugar chain albumin B1 - B25.
Figure 5-1 shows the molecular weights of sugar chain albumin 1 - 125.
Figure 5-2 is a continuation from Figure 5-1.
Figure 5-3 is a continuation from Figure 5-2.
Figure 5-4 is a continuation from Figure 5-3.
Figure 6 shows the fluorescence standard curve of HSA-TAMRA.
Figure 7 shows the results of incubating the sugar chain albumins produced in the present Example together with four types of stomach cancer cell strains (IM-95 (wild-type KRAS gene), KE-39 (KRAS gene amplified form), GSU (KRAS mutated form (G12D)), and SNU-1 (KRAS mutated form (G12D)) at 37°C for 1 hour to carry out priority cell assay. Fluorescence was averaged and normalized from the number of cells (n = 1).

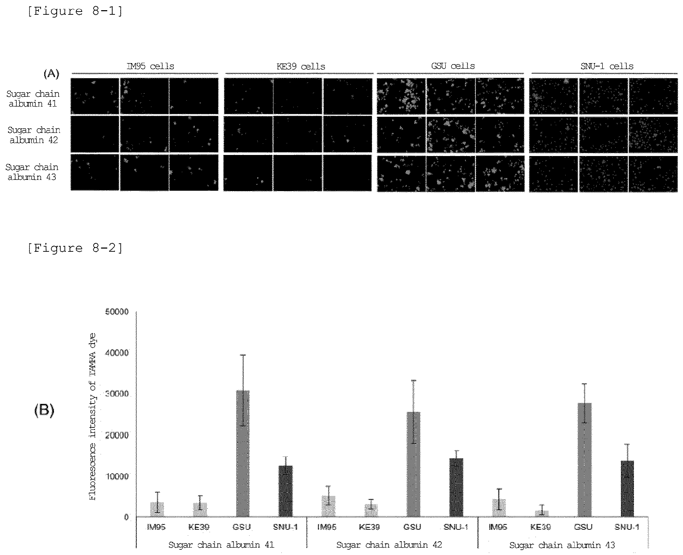

Figure 8 shows the binding priority of sugar chain albumin 41 - 43 against four types of stomach cancer cell strains (IM-95 (wild-type KRAS gene), KE-39 (KRAS gene amplified form), GSU (KRAS mutated form (G12D)), and SNU-1 (KRAS mutated form (G12D)). (A) is the cell imaging result (in triplicates) of IM95, KE39, GSU, and SNU-1 cells incubated with 1 µM of sugar chain albumin 41 - 43 at 37°C for 1 hour. The fluorescence presented is TAMRA fluorescence. (B) shows the comparison of fluorescence intensity observed per cell after incubating IM95, KE39, GSU, and SNU-1 cancer cells with sugar chain albumin 41 - 43. Fluorescence was averaged and normalized from the number of cells (n = 3).
Figure 9 shows the cancer cell growth curve that has the purpose of searching for the effect of either Prodrug 1 or Drug 2 on GSU cell culture (GR50). The GR50 value represents the concentration that gives half of maximum growth rate suppression.

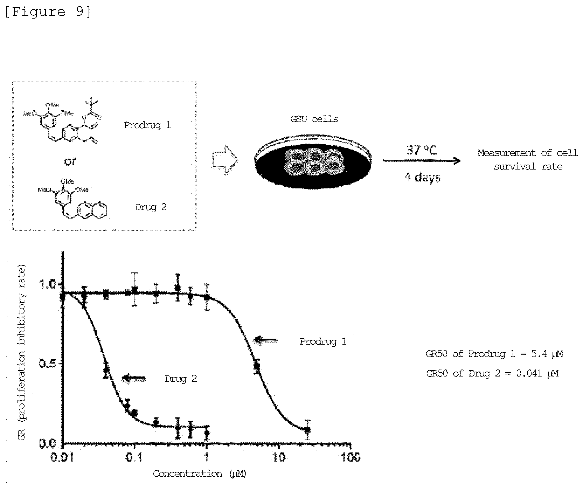

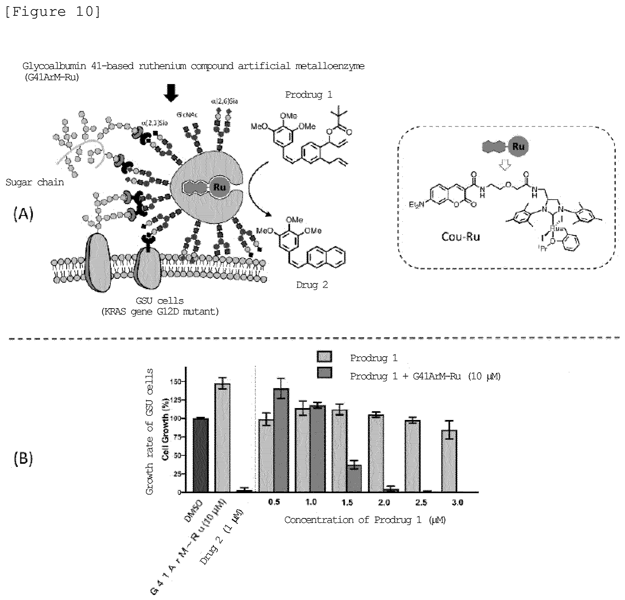

Figure 10 shows the prodrug activating action against GSU cancer cell proliferation by sugar chain albumin 41-based ruthenium compound artificial metalloenzyme (G41ArM-Ru). (A) Schematic diagram of cancer-targeted activation of Prodrug 1 towards anticancer agent 2 employing G41ArM-Ru. (B) Cell toxicity test was performed with GSU cancer cells. In order to determine the concentration of use of Prodrug 1 and agent 2 in this assay, the threshold was investigated first (see Figure 9). Next, cells were treated with various concentrations of Prodrug 1 and 10 µM of G41ArM-Ru. The error bar represents the S.D. of three repeated measurements.

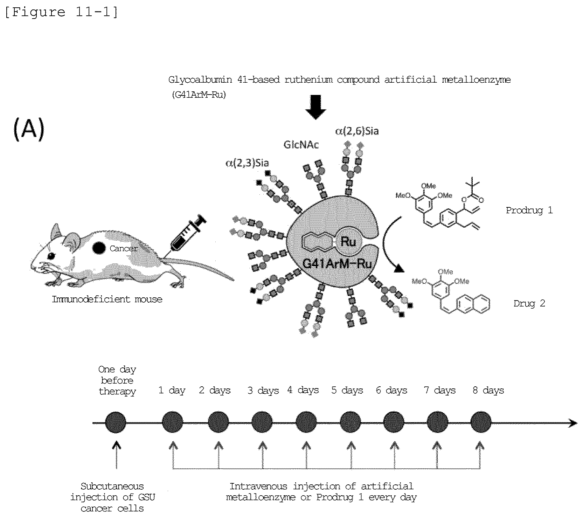

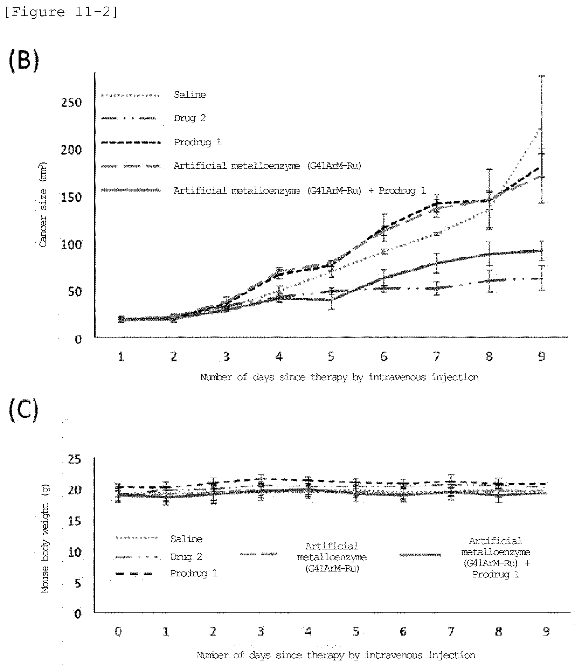

Figure 11 shows the result of *in vivo* prodrug activation by G41ArM-Ru on GSU tumor proliferation of mice. (A) shows the treatment plan of prodrug therapy by G41ArM-Ru by intravenous administration (i.v.) for treating subcutaneous heterotransplanted GSU tumor of mice. Tumor was first transplanted to mice, this was allowed to develop over one day before therapy, and the injection administration was conducted every day over 8 days. The effect of tumor therapy on (B) tumor volume and (C) change in body weight of GSU heterotransplanted mice are shown: saline (n = 4), Drug 2 (33.5 mg/kg, n = 4), Prodrug 1 (75.0 mg/kg, n = 4), G41ArM-Ru (300.0 mg/kg, n = 4), and co-treatment of G41ArM-Ru and Prodrug 1 (300.0 mg/kg and 75.0 mg/kg, n = 4). Data of (B) and (C) are shown with average value ± SD.
Figure 12 shows the imaging results (I - III) in triplet after incubating pancreatic cancer patient-derived organoid (PDO) (RPAN001, mutated KRAS gene (G12D)) with 10 µM of sugar chain albumin 41 at 37°C for 1 hour. The images are observation images of 20x objective lens. Images obtained by fluorescence of TAMRA (1/30s exposure time), fluorescence of DAPI (1/2000s exposure time), bright field (1/4500s exposure time), and superimposed image are shown.
Figure 13 shows the imaging results (I - III) in triplet after incubating pancreatic cancer patient-derived organoid (PDO) (RPAN002, wild-type KRAS gene) with 10 µM of sugar chain albumin 41 at 37°C for 1 hour. The images are observation images of 20x objective lens. Images obtained by fluorescence of TAMRA (1/30s exposure time), fluorescence of DAPI (1/2000s exposure time), bright field (1/4500s exposure time), and superimposed image are shown.
Figure 14 shows the comparison of the binding priority order of sugar chain albumin 41 against two types of pancreatic cancer patient-derived organoids (PDO) (RPAN001 (KRAS mutated form (G12D)) and RPAN002 (wild-type KRAS gene)). A) the result of cell imaging of incubating RPAN001 and (B) RPAN002 with 10 µM of sugar chain albumin 41 at 37°C for 1 hour. The images are observation images of 40x objective lens. Images obtained by fluorescence of TAMRA (1/30s exposure time), fluorescence of DAPI (1/300s exposure time), bright field (1/1100s exposure time), and superimposed are shown.
Figure 15 shows the cancer cell growth curve that has the purpose of searching for the effect of either Prodrug 1 or Drug 2 on pancreatic cancer PDO (RPAN001) culture (GR50). The GR50 value represents the concentration that renders half of maximum proliferation rate suppression.

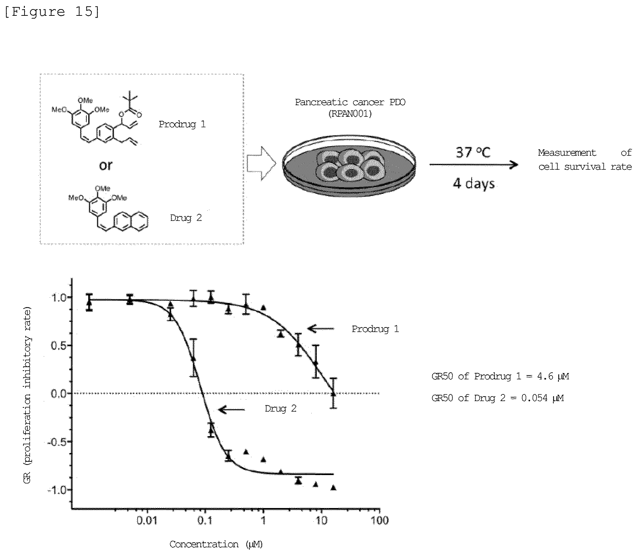

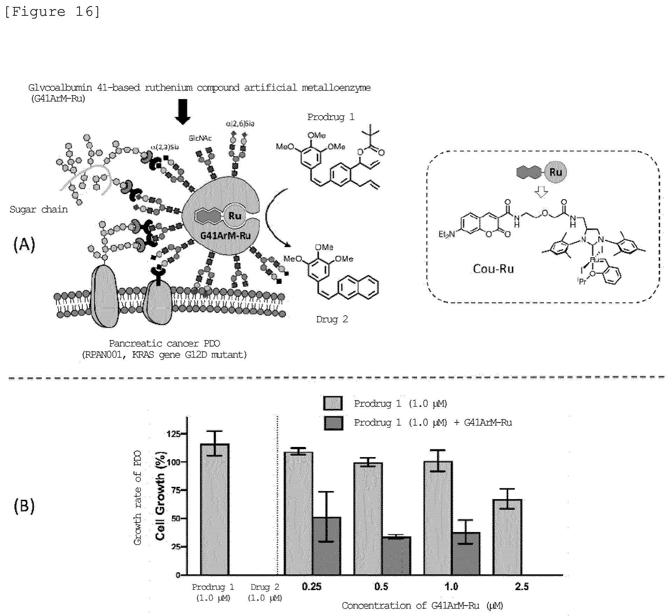

Figure 16 shows the prodrug activation against pancreatic cancer PDO proliferation by G41ArM-Ru. (A) shows the schematic diagram of activation of Prodrug 1 targeting PDO towards anticancer agent 2 by G41ArM-Ru. (B) Pancreatic cancer PDO (RPAN001, mutated KRAS gene (G12D)) was employed to carry out cell toxicity assay. The threshold that would give the maximum difference in activity between Prodrug 1 and Drug 2 was first investigated (1.0 µM for pancreatic cancer PDO (RPAN001), see Figure 15). Then, PDO was treated with various concentrations of G41ArM-Ru and Prodrug 1 (1.0 µM). The error bar represents the S.D. of three repeated measurements.
Figure 17 shows the target selectivity of sugar chain albumin 41 in patient tumor tissue transplantation (Patient-derived xenografts, PDX) model.
Figure 18 shows the result of *in vivo* prodrug activation against pancreatic tumor proliferation of PDX mice having KRAS gene mutation (G12D) by G41ArM-Ru. Data of (B) is shown with average value ± SD.

### Description of Embodiments

The contents of the present disclosure will now be described in detail.

In one aspect, the present disclosure relates to a production method of a protein that selectively recognizes K-ras gene mutation cancer (also referred to below as "the method of the present disclosure").

"K-ras gene" is a type of Ras gene that encodes the RAS protein. There are several K-ras genes that are known to be related to the onset of cancer. In the present disclosure, it refers particularly to the G12D mutant but is not limited thereto, and G12C, G12V, G12A, G12S, G12R, G13D, G13V, G13C, G13R, and the like are also within the scope of the present disclosure. Note that in regard to the notation of mutation in the present disclosure, for example, "G12D" refers to, as is well-known to those skilled in the art, that the 12th codon G of the K-ras gene (such as human K-ras gene) is mutated to D.

In the present disclosure, the cancer type of "K-ras gene mutation cancer" is not particularly limited as long as it is due to a K-ras gene mutation, and for example, can include stomach cancer, gastric adenocarcinoma, pancreatic cancer, colon cancer, lung cancer, endometrial cancer, multiple myeloma, and the like.

In the present disclosure, "selectively recognizes" means that the protein according to the present disclosure binds to K-ras gene mutation cancer with higher affinity than to other tissues, organs, or cells. Accordingly, it does not exclude the protein according to the present disclosure from binding to other tissues, organs, or cells.

In the present disclosure, the protein may be any protein that is capable of modification by a sugar chain, and is typically a protein that has one or more lysine residues suitable for glycosylation on its surface. In a preferred embodiment, the protein in the present disclosure is albumin.

The method of the present disclosure is characterized in that it comprises first to third steps of conjugating first to third sugar chains respectively to lysine residues on the protein molecule.

In the method of the present disclosure, the first sugar chain is (2,6)sialic acid, and is defined by the following structural formula:

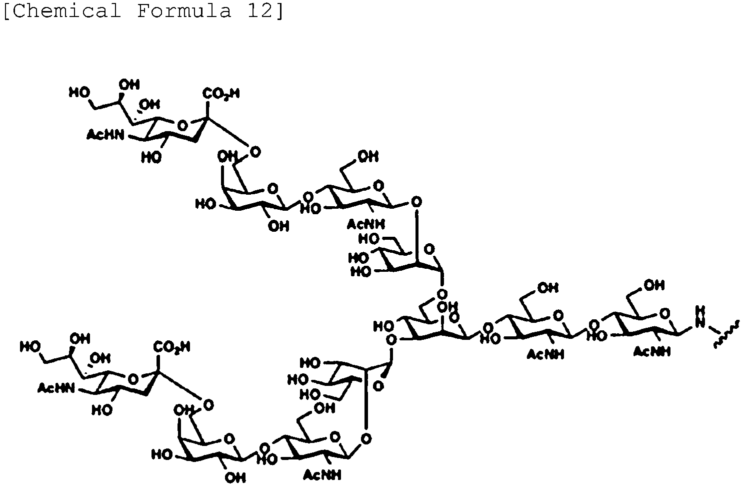

In the method of the present disclosure, the second sugar chain is GlcNAc, and is specified by the following structural formula:

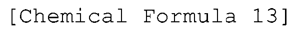

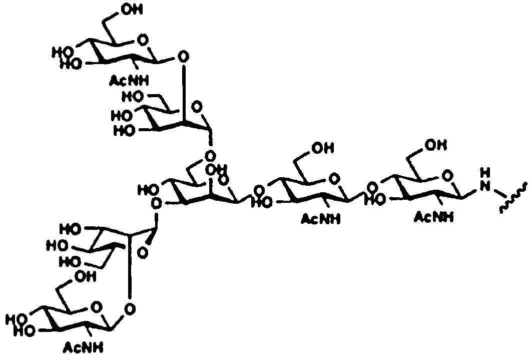

In the method of the present disclosure, the third sugar chain may be any sugar chain, and is typically any of multiantennary complex sugar chains. In the method of the present disclosure, multiantennary complex sugar chains can include, but is not limited to, a doublestranded complex sugar chain, a triple-stranded complex sugar chain, or a quadruple-stranded complex sugar chain, and the like.

In a specific embodiment, the third sugar chain may be any sugar chain that is specified by the following formula: 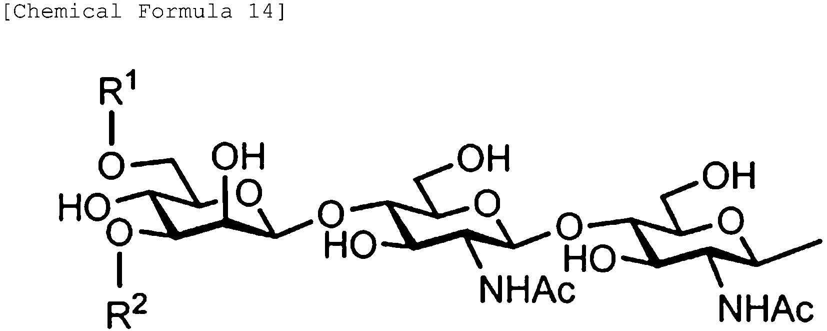 [wherein R¹ and R² are the same or different, and indicate 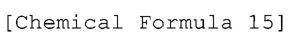 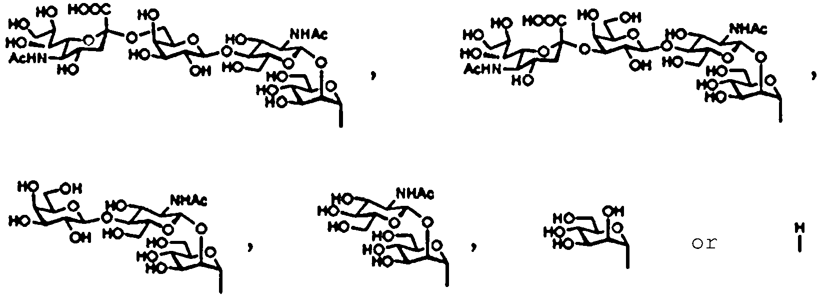 , and Ac indicates an acetyl group.]

The third sugar chain can also be one or multiple types of sugar chains.

In a preferred embodiment, the third sugar chain is selected from sugar chains represented by the following formulae: 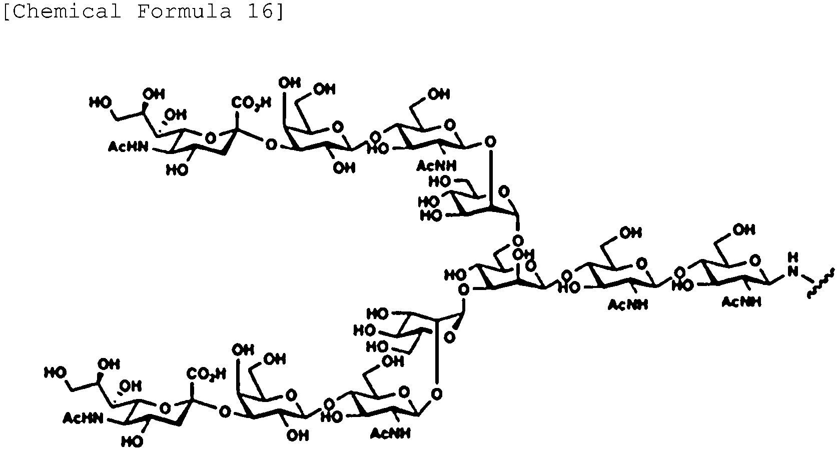 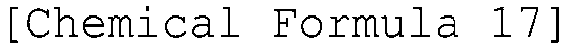 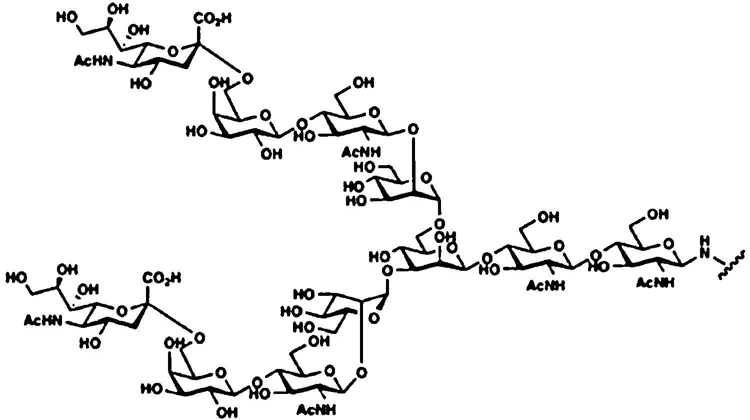 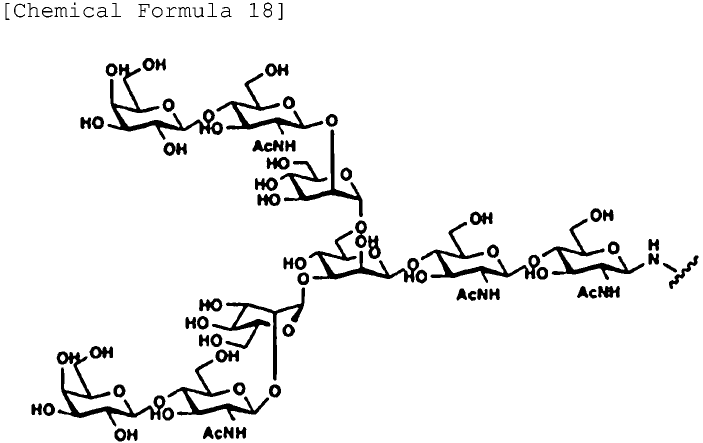 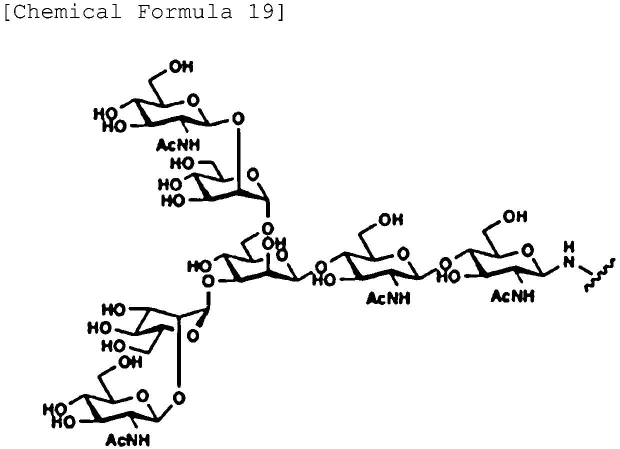 ; and

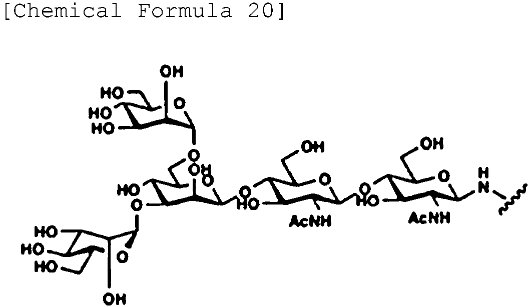

In a further preferred embodiment, the third sugar chain is selected from sugar chains represented by the following formulae: 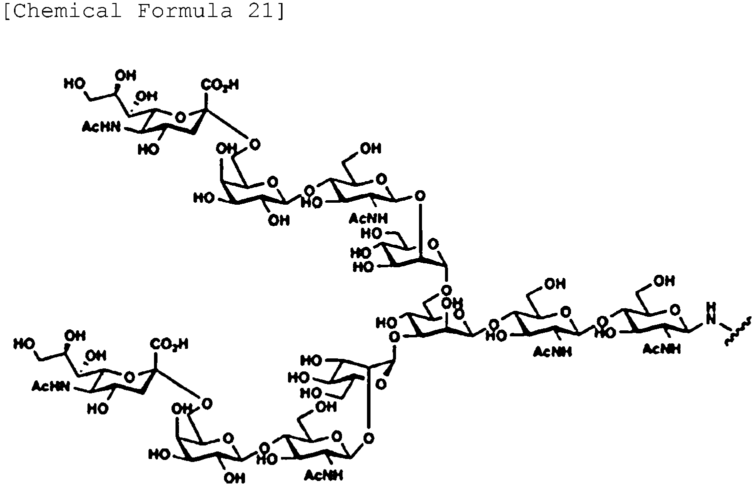 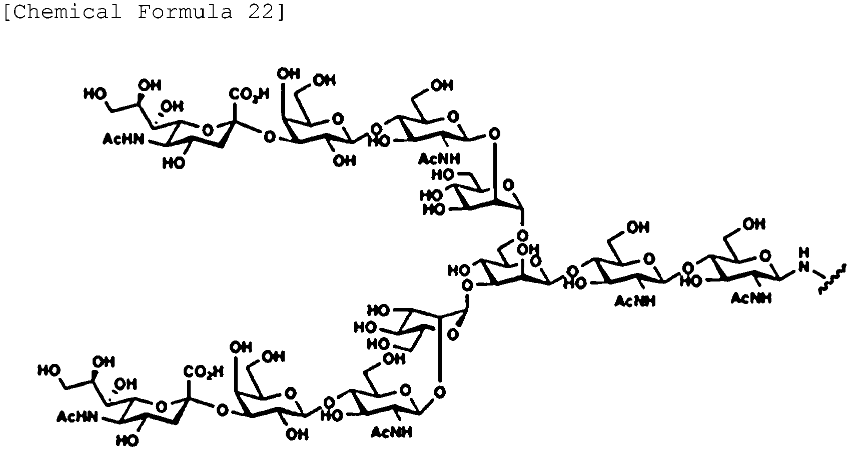 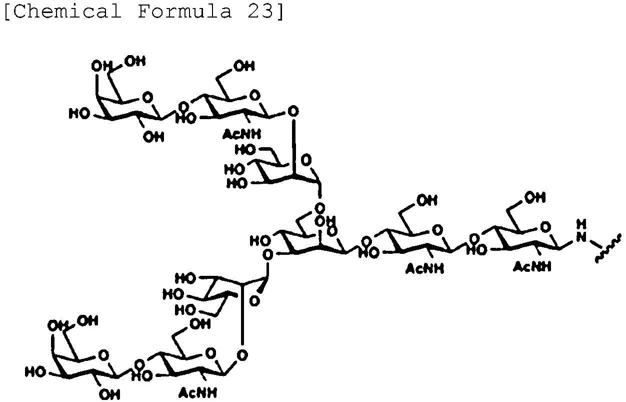 ; and

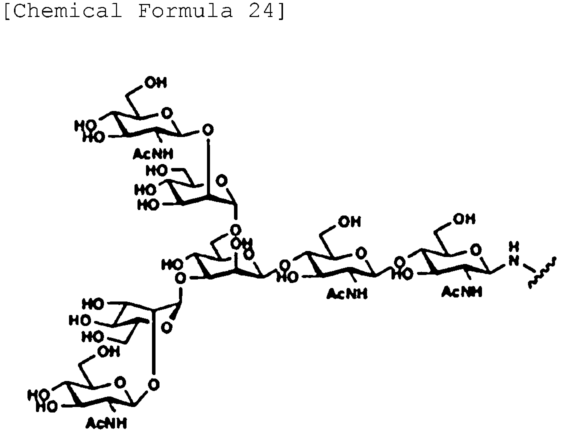

The binding of the sugar chain in the first step is typically performed under conditions such that the first sugar chain will be conjugated to 10 - 30% of the lysine residues present on said protein molecule.

The binding of the sugar chain in the second step is typically performed under conditions such that the second sugar chain will be conjugated to 10 - 30% of the lysine residues present on said protein molecule.

The binding of the sugar chain in the third step is typically performed under conditions such that the third sugar chain will be conjugated to 10 - 30% of the lysine residues present on said protein molecule.

In the method of the present disclosure, by comprising the above configuration, the first, second, and third sugar chains are respectively conjugated in the order of more easily reacted lysines among the lysines present on the protein molecule.

In one embodiment, the protein used is albumin. In the first step, two to five, such as three first sugar chains are conjugated to albumin, in the second step, two to five, such as three second sugar chains are conjugated to albumin, and in the third step, two to five, such as three third sugar chains are conjugated to albumin. Note that the term "about" as used herein may mean comprising the range of plus or minus one or 1% in regard to the mentioned numeric value.

In one embodiment, the protein used is albumin, and a total of 3 - 25, 5 - 20, 7 - 15, or 9 - 11 of the first to third sugar chains are conjugated per molecule. Moreover, in the present disclosure, "a total of 9 - 11 of the first to third sugar chains are conjugated" in regard to multiple proteins according to the present disclosure may mean that 9 - 11 on average of the first to third sugar chains conjugated are conjugated per molecule.

In the present disclosure, the conjugation of sugar chains to lysine residues on the protein molecule can be performed according to previous reports (such as International Publication No. 2017/002918; Katsumasa Fujiki and Katsunori Tanaka, "RIKEN Click Reagent for Protein Labeling", Encyclopedia of Reagents for Organic Synthesis (e-EROS)). Specifically, in the aforementioned means, conjugation to the side chain of lysine residues on the protein molecule is performed employing a sugar chain derivative having a given aldehyde compound added to the carboxyl end of a sugar chain asparagine (e.g. see Figure 1).

The conjugation of the sugar chain derivative and the side chain of the lysine residue can be performed by, for example, mixing both substances in a polar solvent. Polar solvents include, e.g., water, dimethylformamide (DMF), dimethyl sulfoxide (DMSO), cyanidation methyl, propionitrile, diethoxyethane (DME), and mixed solvents thereof, and the like. The reaction is performed preferably at the temperature of 60°C or less, preferably at 50°C or less, and further preferably at 15 - 40°C so that the protein is not denatured.

The proportion/number of sugar chains introduced to one protein molecule can be adjusted by adjusting the molar ratio between the protein and the sugar chain derivative. In other words, the more the amount of the sugar chain derivative is relative to the protein, the higher the proportion/number of molecules of sugar chain introduced to one protein molecule will be achieved. An exemplary molar ratio of introducing about three sugar chains according to the present disclosure per one albumin molecule is 1:8 - 12.

In a preferred embodiment, the protein according to the present disclosure is albumin, and the aforementioned albumin accommodates a metal catalyst in its hydrophobic spot so that it is not exposed or not substantially exposed to the hydrophilic environment. Here, "the catalyst is not substantially exposed to a hydrophilic environment" refers to that exposure to the hydrophilic environment is permitted to the extent that the protection of the catalyst from the hydrophilic environment is recognized. "The protection of the catalyst from the hydrophilic environment is recognized" refers to that the activity of the catalyst accommodated in the protein of the present disclosure is maintained for a longer period of time under the same environment compared to when exposing a free catalyst to an *in vivo* environment, and for example can be evaluated by metabolic turnover (TON).

Note that in the present disclosure, the term "albumin" refers to human serum albumin.

In one embodiment of the present disclosure, "the catalyst is not substantially exposed to a hydrophilic environment" means that the relative solvent accessible surface area (SASA) of the catalyst when accommodated in the hydrophobic spot of albumin is 5.0 or less, preferably 4.0 or less, more preferably 3.5 or less, more preferably 3.0 or less, more preferably 2.5 or less, more preferably 2.0 or less, more preferably, 1.5 or less, more preferably 1.0 or less, more preferably 0.9 or less, more preferably 0.8 or less, more preferably 0.7 or less, more preferably 0.6 or less, more preferably 0.5 or less, more preferably 0.4 or less, more preferably 0.3 or less, more preferably 0.2 or less, or more preferably 0.1 or less.

In another embodiment of the present disclosure, "the catalyst is not substantially exposed to a hydrophilic environment" means that 50% or higher, preferably 55% or higher, more preferably 60% or higher, more preferably 65% or higher, more preferably 70% or higher, more preferably 75% or higher, more preferably 80% or higher, more preferably 85% or higher, more preferably 90% or higher, more preferably 95% or higher, more preferably 96% or higher, more preferably 97% or higher, more preferably 98% or higher, or more preferably 99% or higher of the entire surface area of the catalyst is accommodated inside the hydrophobic spot of albumin. Alternatively, "the catalyst is not substantially exposed to a hydrophilic environment" means that the exposed area of the catalyst to the hydrophilic environment is 50% or lower, preferably 45% or lower, more preferably 40% or lower, more preferably 35% or lower, more preferably 30% or lower, more preferably 25% or lower, more preferably 20% or lower, more preferably 15% or lower, more preferably 10% or lower, more preferably 5% or lower, more preferably 4% or lower, more preferably 3% or lower, more preferably 2% or lower, or more preferably 1% or lower of the entire surface area of the catalyst.

In the present disclosure, the hydrophobic spot of albumin can include albumin drug binding site I and albumin drug binding site II. In one embodiment, the protein of the present disclosure accommodate the metal catalyst in albumin drug binding site I.

The metal catalyst that can be used in the albumin of the present disclosure is not particularly limited, and can be arbitrarily selected by those skilled in the art. In one embodiment, the metal catalyst that can be used in the albumin of the present disclosure is one that has the activity to change a given prodrug to active.

For example, the metal catalyst that is used in the albumin of the present disclosure can include, but is not limited to, a boron catalyst, a magnesium catalyst, an aluminum catalyst, a silicon catalyst, a calcium catalyst, a scandium catalyst, a titanium catalyst, a vanadium catalyst, a chromium catalyst, a manganese catalyst, an iron catalyst, a cobalt catalyst, a nickel catalyst, a copper catalyst, a zinc catalyst, a yttrium catalyst, a zirconium catalyst, a niobium catalyst, a molybdenum catalyst, a ruthenium catalyst, a rhodium catalyst, a palladium catalyst, a silver catalyst, a indium catalyst, a tin catalyst, a barium catalyst, a hafnium catalyst, a tungsten catalyst, a rhenium catalyst, a osmium catalyst, a iridium catalyst, a platinum catalyst, a gold catalyst, and a lanthanoid Lewis acid catalyst, and the like. Moreover, said lanthanoid Lewis acid catalyst can include, but is not limited to, an ytterbium catalyst, a lanthanum catalyst, a cerium catalyst, a samarium catalyst, a europium catalyst, a gadolinium catalyst, a terbium catalyst, a thulium catalyst, and a lutetium catalyst, and the like.

The means for accommodating the catalyst in the hydrophobic spot of albumin can include, e.g. means to bind the catalyst inside the hydrophobic spot via a ligand that interacts or binds to said hydrophobic spot. In this case, for the linking of the ligand and the catalyst (such as metal catalyst), a linker such as peptide, hydrocarbon chain, PEG, and the like can be appropriately employed. The length of the linker used should be a length such that the catalyst is accommodated inside the hydrophobic spot, and not exposed or not substantially exposed to the hydrophilic environment. In other words, if the linker used is too long for the sizes of the hydrophobic spot, the ligand that interacts therewith, and the catalyst used, there is a possibility that when binding the catalyst to the hydrophobic spot via the linker, the catalyst is not housed within the hydrophobic spot, a portion or the entirety thereof is exposed from the hydrophobic spot to the hydrophilic environment, and sufficient protection against the hydrophilic environment is not obtained.

Ligands that can be used for this purpose may change depending on the hydrophobic spot that accommodates the metal catalyst. For example, when the metal catalyst is accommodated in albumin drug binding site I, said ligand can be selected from warfarin, azapropazone, acenocoumarol, phenylbutazone, salicylate salt, indomethacin, phenytoin, tolbutamide, chlorpropamide, iophenoxate, iodipamide, sulfadimethoxine, phenprocoumon, glibenclamide, sulfathiazole, tenoxicam, camptothecin, prodan, bilirubin, eicosanoid, and carboxy-methyl-propyl-furan propanoate (uremic toxin), and coumarin. Alternatively, when the metal catalyst is accommodated in albumin drug binding site II, said ligand can be selected from diazepam, ketoprofen, chlofibrate, ibuprofen, iopanoate, azide deoxythymidine, flufenamate, ethacrynate, naproxen, flurbiprofen, cicloprofen, benoxaprofen, flucloxacillin, chlorothiazide, pirprofen, propofol, isoflurane, dansylsarcosine, dansylglycine, alkylaminocoumarin acetic acid, hydroxyflavone, L-tryptophan, medium chain fatty acid anion (such as octanoate), L-thyroxine, chloride ion, iodoacetic acid, indoxyl sulfate, and hippuric acid (urotoxin). In one embodiment of the present disclosure, the hydrophobic spot of albumin is drug binding site I, and a coumarin derivative such as 7-dimethylamino coumarin is used as the ligand in order to accommodate ruthenium in the aforementioned site.

The linking of the metal catalyst and said ligand may be performed via a linker. Linkers that can be used for this purpose are typically an alkyl chain or a polyethylene glycol (PEG) chain that has an amino group and a carboxy group at both ends, and the like. Specifically, alkyl chain linkers can include, for example -NH-(CH₂)ₓ-CO- (wherein x is an integer, and although not limited as long as it does not inhibit the intended linker function, it can be e.g. an integer from 1 - 15), -NH-(CH₂CH₂O)_{y}-CH₂-CO- (wherein y is an integer, and although not limited as long as it does not inhibit the intended linker function, it can be e.g. an integer from 1 - 15), or -NH-(CH₂CH₂O)_{z}-CH₂CH₂-CO- (wherein z is an integer, and although not limited as long as it does not inhibit the intended linker function, it can be e.g. an integer from 1 - 15), and the like.

When a linker is employed for the linking of the metal catalyst and said ligand, it should be noted that the length of the linker used is a length that the metal catalyst is accommodated inside the hydrophobic spot of albumin, and not exposed or not substantially exposed to the hydrophilic environment. For example, when coumarin or a derivative thereof (such as 7-dimethylamino coumarin, 7-diethylamino coumarin (DEAC)) is employed as the ligand to accommodate ruthenium in albumin drug binding site I, "not substantially exposed to a hydrophilic environment" may be that the exposed area of ruthenium to the hydrophilic environment is 40% or lower, preferably 35% or lower of the entire surface area of ruthenium. Alternatively, "not substantially exposed to a hydrophilic environment" may be that SASA of ruthenium when accommodated in albumin drug binding site I is 3.0 or less, preferably 1.0 or less. Moreover, an exemplary linker that is used therefor is - NH-(CH₂CH₂O)_{y}-CH₂-CO-, wherein y is an integer from 1 to 6, preferably an integer from 1 to 3.

Another means for accommodating the catalyst in the hydrophobic spot of albumin can include, e.g. means for allowing maleimide or succinimide to act on and activate the side chain functional group of cysteine or lysine (thiol group and amino group, respectively) that is present inside or in the vicinity of the hydrophobic pocket, to covalently bind to the catalyst.

Yet another means for accommodating the catalyst in the hydrophobic spot of albumin can include means for introducing a mutation at a particular position in the amino acid sequence configuring the hydrophobic pocket to introduce a double bond or an azide, and performing metathesis or Click reaction to allow covalent binding to the catalyst (Young TS, Ahmad I, Brock A, Schultz PG., Expanding the genetic repertoire of the methylotrophic yeast Pichia pastoris., Biochemistry. 2009. 48 (12): 2643-53.; Wang L, Schultz PG., Expanding the genetic code., Angew Chem Int Ed Engl. 2004. 44 (1): 34-66.). In this case, a catalyst having a linker such as a peptide or PEG can be used as the metal catalyst.

The protein of the present disclosure produced by the method of the present disclosure is one having rendered tropism towards K-ras gene mutation cancer. Accordingly, in another aspect, the present disclosure relates to a pharmaceutical composition for cancer therapy comprising the protein of the present disclosure.

In one embodiment, the pharmaceutical composition of the present disclosure is a pharmaceutical composition used in combination with a prodrug that may be activated by the protein of the present disclosure. In the aforementioned embodiment, the pharmaceutical composition of the present disclosure may be in a single-drug form comprising a pharmaceutically acceptable carrier, and in some cases further comprising a prodrug that may be activated by the protein of the present disclosure. Prodrugs that may be activated by the protein of the present disclosure that can be used in the present disclosure can include e.g. various anticancer agents, and can be illustrated by, but not limited to, specifically, mitomycin C, doxorubicin, taxol, endoxifen, and the like.

In another embodiment, the pharmaceutical composition of the present disclosure is in a form of combination medicine where the protein of the present disclosure and a prodrug that may be activated by the aforementioned protein are provided as separate agents. In the combination medicine of the present disclosure, a first agent comprising the protein of the present disclosure and a second agent comprising a prodrug that may be activated by the aforementioned protein can be administered to a subject at the same or different times.

In another aspect, the pharmaceutical composition of the present disclosure relates to a pharmaceutical composition comprising a prodrug that may be activated by the protein of the present disclosure and any pharmaceutically acceptable carrier, and the aforementioned pharmaceutical composition can be characterized by being used in combination with the protein of the present disclosure.

Note that the terms used herein are to be employed to describe particular embodiments, and do not intend to limit the invention.

Moreover, the term "comprising" as used herein, unless the content clearly indicates to be recognized otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meaning as that broadly recognized by those skilled in the art of the technology to which the present disclosure belongs. The terms used herein, unless explicitly defined otherwise, should be construed as having meanings consistent with the meanings herein and in related technical fields, and shall not be construed as having idealized or excessively formal meanings.

Terms such as first and second may be employed to express various elements, and it should be recognized that these elements are not to be limited by these terms. These terms are employed solely for the purpose of discriminating one element from another, and for example, it is possible to describe a first element as a second element, and similarly, to describe a second element as a first element without departing from the scope of the present disclosure.

The present disclosure will now be more specifically described by Examples. However, the present disclosure can be embodied by various embodiments, and shall not be construed as being limited to the Examples described herein.

### Example 1

### Preparation of nonuniform sugar chain albumin

### 1.1 Materials and instruments

General reagents and buffer components were purchased from Sigma-Aldrich, Fisher Scientific, Alfa Aesar, TCI, or Wako Chemicals. Fmoc-protected complex N-glycan was provided from Glytech Inc. Human serum albumin (lyophilized powder) was purchased from Sigma-Aldrich (Prod # A1653). Amicon^{™} Ultra Centrifugal Filter (30 kDa) was purchased from Merck Millipore (Burlington, USA). Ultrapure water was obtained from Merck Millipore (Burlington, USA, Milli-Q Advantage^{™} A10 Water Purification System). Matrix-assisted laser desorption-ionization (MALDI-TOF) mass spectrometry was performed on Bruker autoflex spectrometer^{™} with 3,5-dimethoxy-4-hydroxycinnamic acid (sinapic acid) as the matrix.

### 1.2 Preparation of sugar chain-aldehyde compound

Figure 1 shows the flow of the synthetic method of sugar chain albumin that can be employed in the present disclosure.

In the present Example, the sugar chain-aldehyde compound was prepared according to the following general procedure.

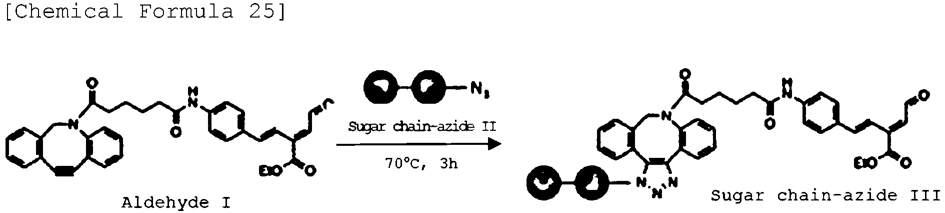

### Synthesis of sugar chain-aldehyde compound

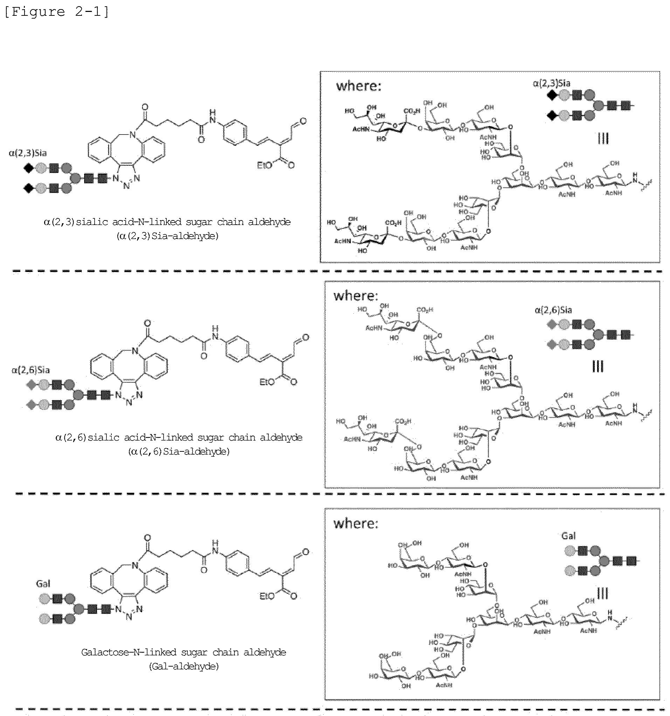

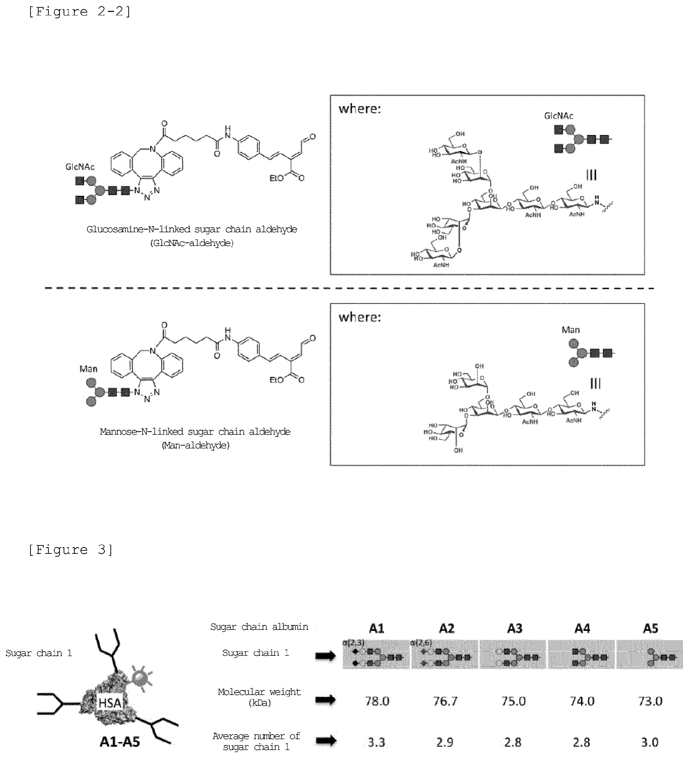

Aldehyde I and sugar chain-azide II were prepared with the procedure of a previous report (Sibgatullina, R., et al., Tetrahedron Lett. 58,1929-1933 (2017)). To a solution (90 µL) of sugar chain-azide II (0.50 µmol) in dimethyl sulfoxide (DMSO) was added aldehyde I (0.45 µmol, 10 mM stock solution in acetonitrile (MeCN), 45 µL). The mixture obtained was incubated under nitrogen atmosphere at 70°C for 3 hours. The mixed solution was cooled to room temperature (r.t.), and MeCN was removed under rotary evaporation to obtain sugar chain-aldehyde III (5.0 mM stock solution in DMSO). The entire chemical structure of the sugar chain-aldehyde compound used in the present Example is shown in Figure 2.

### 1.3 Preparation of TAMRA-labeled human serum albumin (HSA)

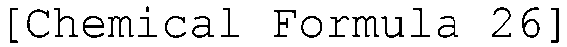

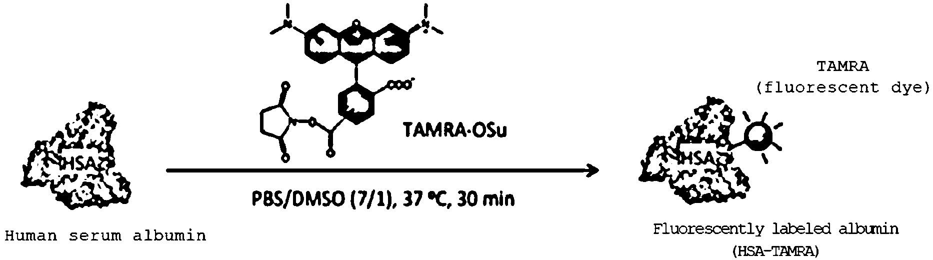

### Synthesis of HSA-TAMRA

As shown above, to a solution of human serum albumin (HSA, 33.3 mg, 500 nmol) in phosphate buffer (pH = 7.4, 437 µL) was added TAMRA-OSu (0.66 mg, 1250 nmol) in DMSO (63 µL), and this was heated to 37°C. After incubating for 30 minutes, the solution was centrifuged with Amicon^{™} Ultra Centrifugal Filter (30 kDa cutoff) to filter off the low molecular weight. The residue was further washed with water, and centrifugation was performed 4 times. The solution obtained was diluted with ultrapure water to 1.0 µL to obtain the stock solution of HSA-TAMRA (500 µM). This stock solution was used for subsequent N-glycan modification. It was detected by MALDI-TOF-MS (positive mode) that the molecular weight of HSA-TAMRA was 67.9 kDa and that it comprises on average 2 TAMRA fluorescent substances per albumin molecule.

### 1.4 Preparation of 125 types of heterogenous sugar chain albumins

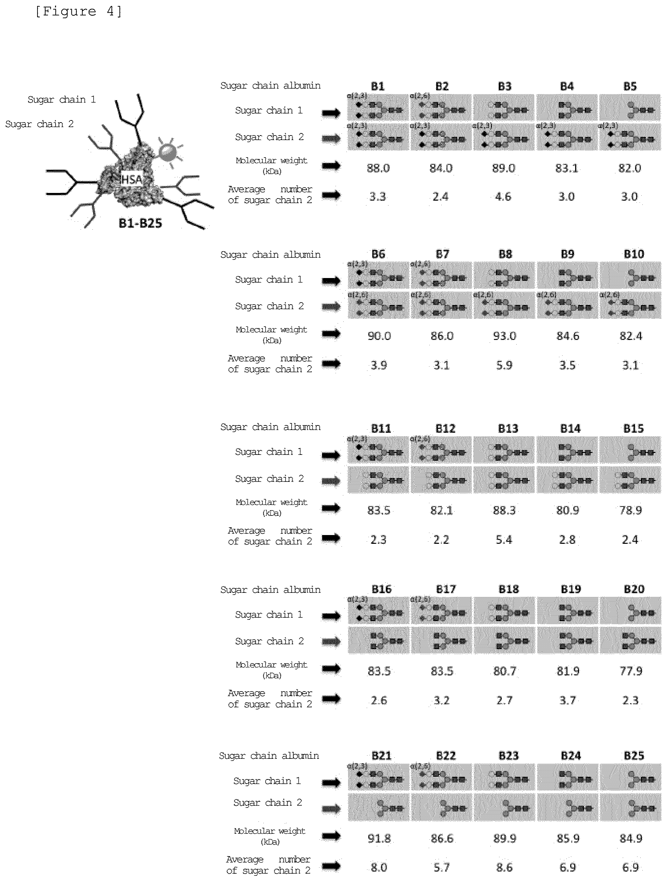

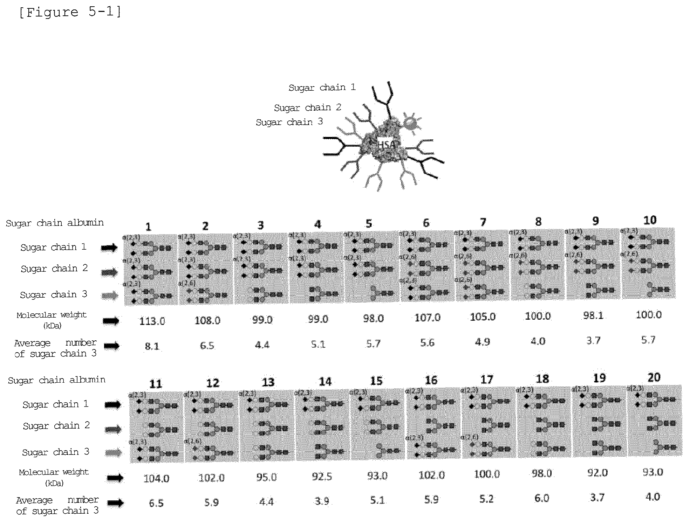

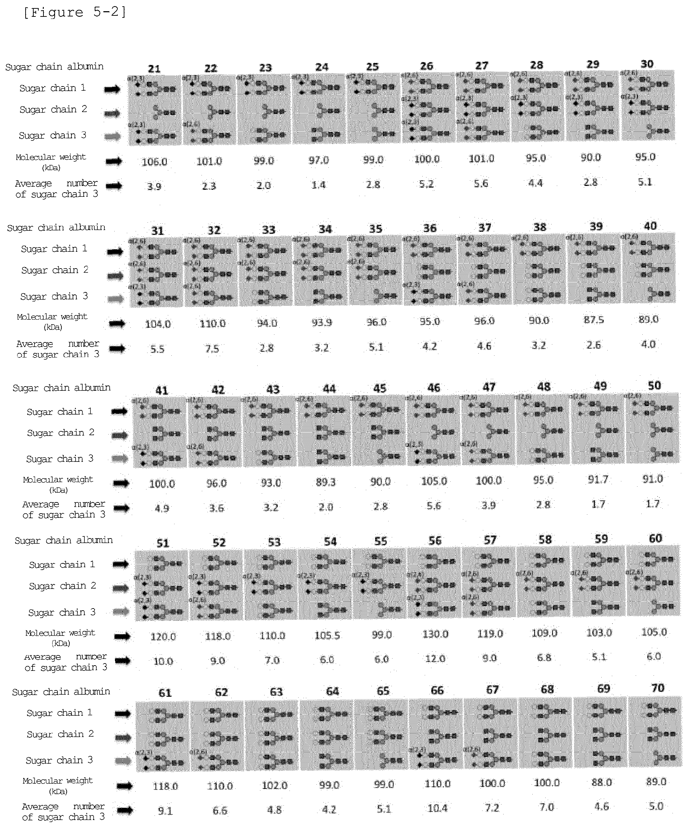

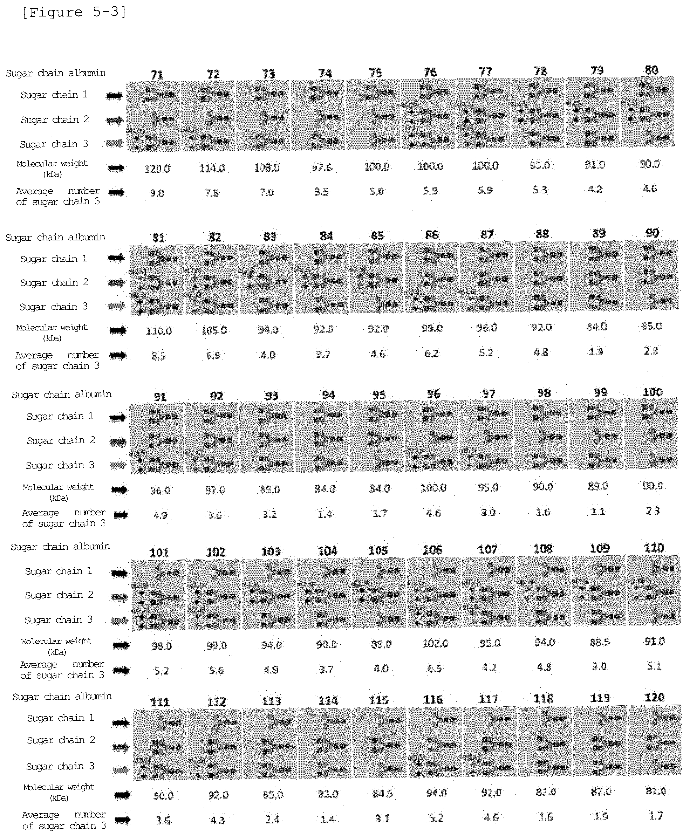

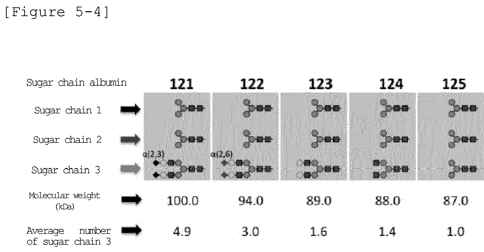

In the synthetic scheme of 125 types of heterogenous sugar chain albumins, preparation was carried out in 3-stage order. First, HSA-TAMRA was treated with 5 types of sugar chain aldehydes (α(2,3)Sia-, α(2,6)Sia-, Gal-, GlcNAc-, and Man-aldehyde) to generate intermediate sugar chain albumin A1 - A5 (Figure 3). Next, A1 - A5 were treated with 5 types of sugar chain aldehydes (α(2,3)Sia-, α(2,6)Sia-, Gal-, GlcNAc-, and Man-aldehyde) to generate intermediate sugar chain albumin B1 - B25 comprising 2 types of N-glycans (Figure 4). Finally, B1 - B25 were once again treated with 5 types of sugar chain aldehydes (α(2,3)Sia-, α(2,6)Sia-, Gal-, GlcNAc-, and Man-aldehyde) to generate 125 types of sugar chain albumin 1 - 125 comprising 3 types of N-glycans as shown in Figures 5-1 to 5-4.

### 1.4.1 General protocol of sugar chain albumin A1 - A5 synthesis

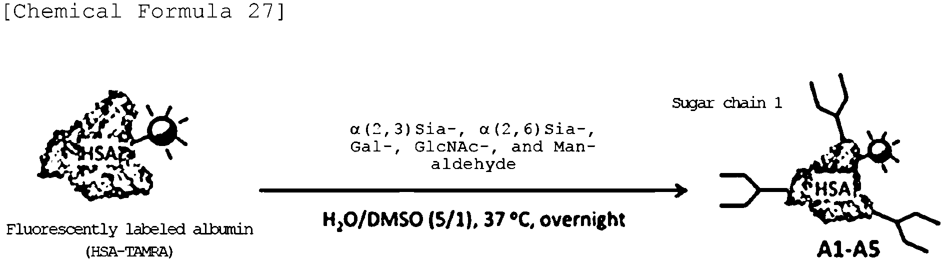

### Synthesis of sugar chain albumin A1 - A5

In a representative example, the reaction mixture was composed of HSA-TAMRA (180 nmol, 0.5 mM stock solution in H₂O, 360 L) and 8 equivalents of α(2,3)Sia-aldehyde (1440 nmol, 5 mM stock solution in DMSO, 288 µL). The total reaction volume was adjusted to give 2.16 mL of 16.6% DMSO in H₂O. This solution was gently mixed, and incubated at 37°C overnight. The solution was then concentrated, and washed with H₂O with Amicon^{™} Ultra Centrifugal Filter (30 kDa cutoff). The insoluble byproduct was filtered off with Durapore PVDF 0.45 µm^{™}, diluted to 0.4 ml with ultrapure water to give the stock solution of sugar chain albumin A1, and this was used for subsequent N-glycan modification. It was confirmed by MALDI-TOF-MS (positive mode) that the molecular weight of sugar chain albumin A1 was 78.0 kDa and that it comprises on average 3.3 α(2,3)Sia-N-glycans per albumin molecule.

### 1.4.2 Overview of synthetic protocol of sugar chain albumin B1 - B25

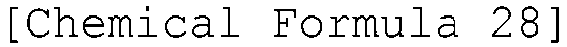

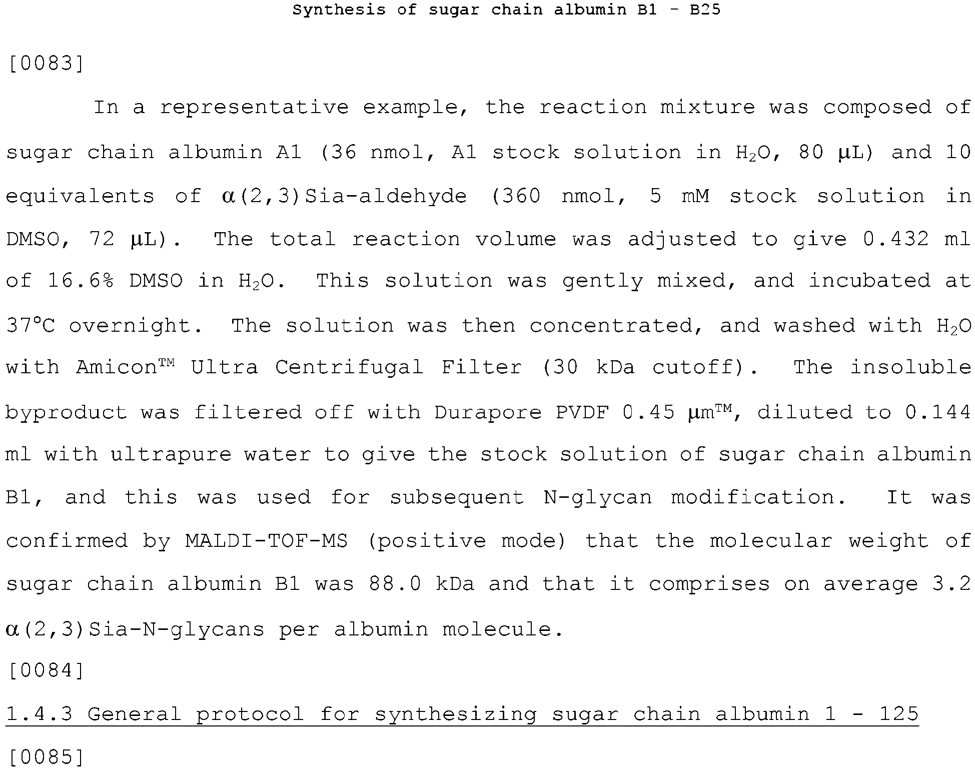

### Synthesis of suger chain albumin B1-B25

In a representative example, the reaction mixture was composed of sugar chain albumin A1 (36 nmol, A1 stock solution in H₂O, 80 µL)and 10 equivalents of a(2,3) Sia-aldehyde (360 nmol, 5 mM stock solution in DMSO, 72 µL). The total reaction volume was adjusted to give 0.432 ml of 16.6% DMSO in H₂O. This solution was then concentrated, and washed with H₂O with Amicon^{™} Ultra Centrifugal Filter (30 kDa cutoff). The insoluble byproduct was filtered off with Durapore PVDF 0.45 µL^{™}, diluted to 0.144 ml, with ultrapure water to give the stock solution of sugar chain albumin B1, and this was used for subsequent N-glycan modification. It was confirmed by MALDI-TOF-MS (positive mod) that the molecular weight of sugar chain alnumin B1 was 88.0 kDa and that it comprises on avarage 3.2 a(2,3) Sia-N-glycans per albumin molecule.

### 1.4.3 General protocol for synthesizing sugar chain albumin 1-125

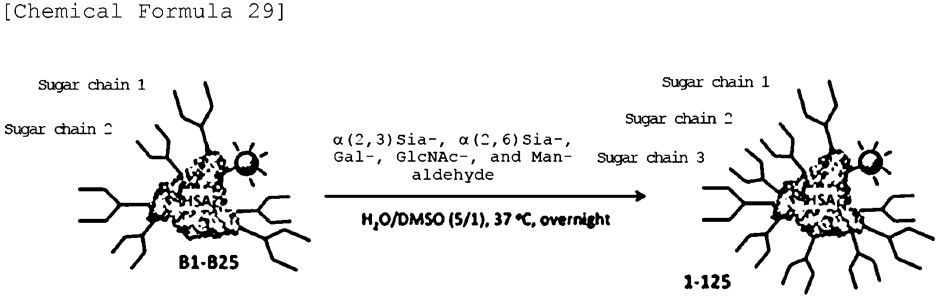

### Synthesis of sugar chain albumin 1 - 125

As a representative example, sugar chain albumin B1 (7.0 nmol, B1 stock solution in H₂O, 28 µL) and 12 equivalents of α(2,3)Sia-aldehyde (84 nmol, 5 mM stock solution in DMSO, 16.8 µL) were used as the reaction mixture. The total reaction volume was adjusted to secure 86 µL of the stock solution of 16.6% DMSO in H₂O. This solution was gently mixed, and incubated at 37°C overnight. This solution was concentrated, and washed with H₂O with Amicon^{™} Ultra Centrifugal Filter (30 kDa cutoff). It was found by MALDI-TOF-MS (positive mode) that the molecular weight of sugar chain albumin 1 was 113.0 kDa and that it comprises on average 8.1 α(2,3)Sia-N-glycans per albumin molecule.

### 1.4.4 Bulk synthetic protocol of sugar chain albumin 41 without TAMRA

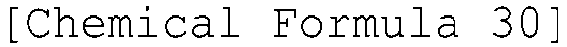

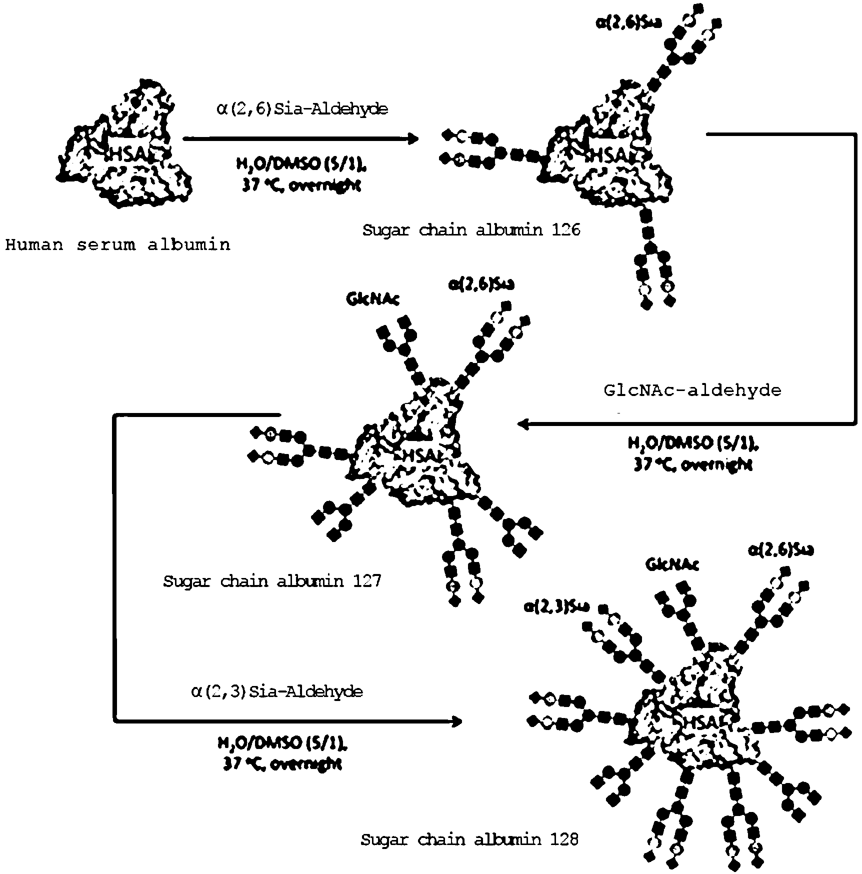

### Synthesis of sugar chain albumin 41 (sugar chain albumin 128) without TAMRA

The reaction mixture was composed of HSA (2400 nmol, 0.5 mM stock solution in H₂O, 4.8 ml) and 10 equivalents of α(2,6)Sia-aldehyde (24000 nmol, 8 mM stock solution in DMSO, 3.0 ml). The total reaction volume was adjusted in order to give 18.0 ml of 16.6% DMSO aqueous solution. This solution was gently mixed, and incubated at 37°C overnight. This solution was concentrated, and washed with H₂O with Amicon^{™} Ultra Centrifugal Filter (30 kDa cutoff). The insoluble byproduct was filtered off with Durapore PVDF 0.45 µm^{™}, diluted to 4.8 ml with ultrapure water to give sugar chain albumin 126, and this was used for subsequent N-glycan modification. It was detected by MALDI-TOF-MS (positive mode) the molecular weight of glycoalbumin 126 was 75.0 kDa and that it comprises on average 2.8 α(2,6)Sia-N-glycans per albumin molecule.

Next, a reaction mixture consisting of sugar chain albumin 126 (2400 nmol, 0.5 mM stock solution in H₂O, 4.8 ml) and 10 equivalents of GlcNAc-aldehyde (24000 nmol, 8 mM stock solution in DMSO, 3.0 ml) was prepared. The total reaction volume was adjusted to give 18.0 ml of 16.6% DMSO aqueous solution. This solution was gently mixed, and incubated at 37°C overnight. This solution was concentrated, and washed with H₂O with Amicon^{™} Ultra Centrifugal Filter (30 kDa cutoff). The insoluble byproduct was filtered off with Durapore PVDF 0.45 µm^{™}, diluted to 4.8 ml with ultrapure water to give the stock solution of sugar chain albumin 127, and this was used for subsequent N-glycan modification. It was confirmed by MALDI-TOF-MS (positive mode) that the molecular weight of sugar chain albumin 127 was 82.4 kDa and that it comprises on average 3.5 GlcNAc-N-glycans per albumin molecule.

Finally, a reaction mixture consisting of sugar chain albumin 127 (2400 nmol, 0.5 mM stock solution in H₂O, 4.8 ml) and 10 equivalents of α(2,3)Sia-aldehyde (24000 nmol, 8 mM stock solution in DMSO, 3.0 ml) was prepared. The total reaction volume was adjusted to give 18.0 ml of 16.6% DMSO aqueous solution. This solution was gently mixed, and incubated at 37°C overnight. This solution was concentrated, and washed with H₂O with Amicon^{™} Ultra Centrifugal Filter (30 kDa cutoff). The insoluble byproduct was filtered off with Durapore PVDF 0.45 µm^{™}, diluted to 4.8 ml with ultrapure water to give the stock solution of sugar chain albumin 128, and this was used for subsequent N-glycan modification. It was confirmed by MALDI-TOF-MS (positive mode) that the molecular weight of sugar chain albumin 128 was 96.0 kDa and that it comprises on average 4.4 α(2,3)Sia-N-glycans per albumin molecule.

### 1.4.5 Measurement of sugar chain albumin concentration

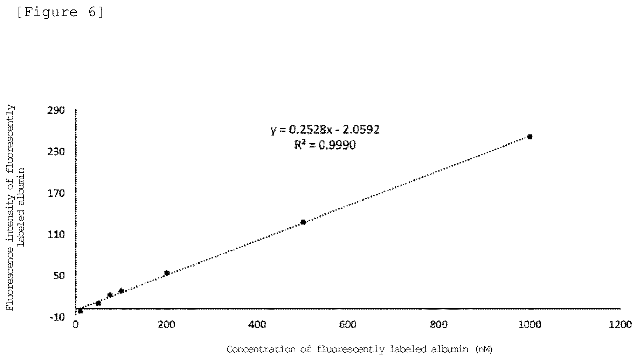

In order to determine the concentration of the sugar chain albumin synthesized, HSA-TAMRA at various concentrations was employed to create a standard curve (Figure 6). The fluorescence intensity of TAMRA was measured at λEX = 530 - 550 nm/λEM = 575 nm with SpectraMax^{™} iD3 Multi-Mode Microplate Reader from Molecular Devices.

### Example 2

### 2. Tests employing cells

### 2.1 General tissue culture condition

In this research, the following cell strains were obtained from RIKEN Cell Bank. The cells were cultured under 5% CO₂ gas atmosphere at 37°C. The media used were as follows.

**[Table 1]**

| Name | Type | Medium | FBS | *KRAS* gene mutation | Penicillin-streptomycin |
|---|---|---|---|---|---|
| IM95 | Human gastric adenocarcinoma cell | DMEM (high glucose) w/ L-glutamine and Phenol Red | 10% | Wild-type | 1% |
| KE-39 | Human gastric adenocarcinoma cell | RPMI1640 | 10% | Amplified | 1% |
| GSU | Human gastric adenocarcinoma cell | RPMI1640 | 10% | G12D | 1% |
| SNU-1 | Human gastric adenocarcinoma cell | RPMI1640 | 10% | G12D | 1% |

### 2.2 Protocol of binding assay of sugar chain albumin to cells

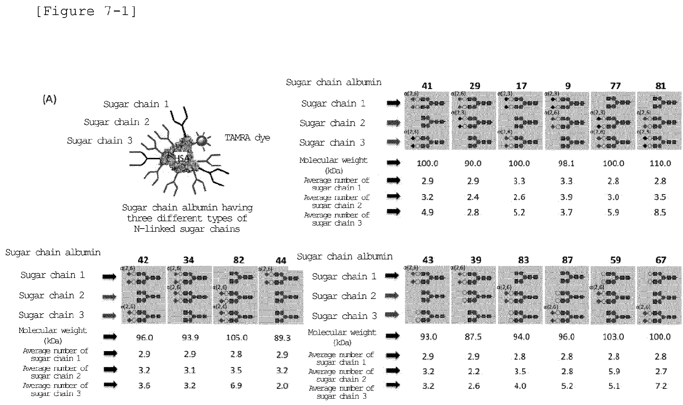

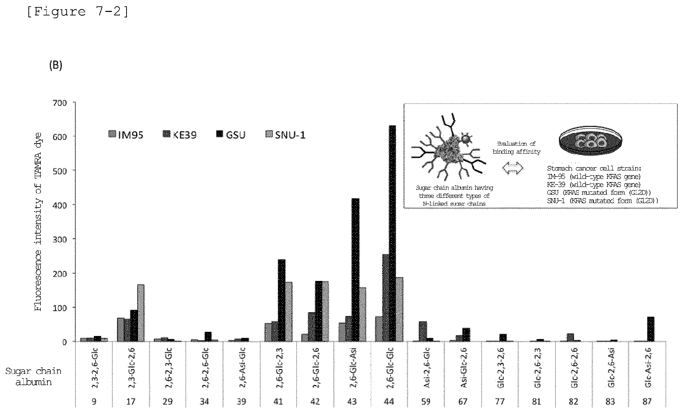

After collecting the cells from the cell dish, the cells were transferred to a 96-well plate at a density of 1 x 10⁴ cells/46 µl of medium. Stock solutions of sugar chain albumin 1 - 125 and HSA-TAMRA were prepared at a concentration of 13 µM in H₂O. In order to initiate incubation, to 46 µl of the cell solution, 4 µl of each sugar chain albumin was added (final concentration was 1 µM sugar chain albumin/well) . As control, a solution comprising HSA-TAMRA only was incubated at the same concentration, and water was used as negative control. The cells were then incubated at 37°C for 1 hour. The cells per well were then transferred to a 1.5 ml Eppendorf tube, and after 5 minutes of centrifugation at 200 x g, washed 3 times with PBS buffer (200 µl, 4°C). Nucleus enhancer Hoechst (500-fold diluted solution) was added to the cells, and after incubating at 4°C for 5 minutes, 4% paraformaldehyde (100 µl) was added. After incubation at room temperature for 5 minutes, the cells were washed once with PBS buffer (200 µl), and resuspended in PBS buffer (50 µl). Finally, the cells were transferred to a 96-well plate to prepare for microscopic observation with a fluorescence microscope BZ-X710 (Keyence, Tokyo, Japan). Observation of TAMRA dye was measurement of fluorescence at λEX = 530 - 550 nm/λEM = 575 nm, and observation of Hoechst dye at λEX = 330 - 385 nm/λEM = 460 nm. Images were observed and photographed at a magnification of 40x. The fluorescence value was analyzed with ImageJ, the number of cells counted were averaged, and the value for water was subtracted from all data. The combined results of the binding affinity of sugar chain albumin 1 - 125 and four types of stomach cancer cells (IM95, KE-39, GSU, and SNU-1) are shown in Figure 7 and Figure 8.

### 2.3 Protocol of prodrug therapy by G41ArM-Ru against GSU cells

### 2.3.1 Preparation protocol of G41ArM-Ru

The reaction mixture was composed of Cou-Ru (65 nmol, 100 µL from 650 µM stock solution in dioxane) and sugar chain albumin solution: sugar chain albumin 128 (65 nmol, 280 µL from 235 µM stock solution in H₂O). The total reaction volume was appropriately adjusted so that 1000 µL of 10% dioxane in PBS buffer pH 7.4 was secured. After initiation by addition of sugar chain albumin, the reaction mixture was gently mixed, and incubated at 37°C for 1 hour. The solution was then concentrated with Amicon^{™} Ultra Centrifugal Filter (30 kDa cutoff) and washed with PBS buffer. The G41ArM-Ru solution was diluted with PBS buffer as necessary to the necessary amount. The amount for obtaining 1000 µM of stock solution was set at 65 µL.

### 2.3.2 Protocol of cell survival rate measurement

Cell survival rate was measured by employing MTS assay which monitors the reduction of MTS tetrazolium salt into formazan via mitochondrial dehydrogenase of cells with high metabolic activity. The commercially available kit used in this research was CellTiter 96^{™} AQueous One Solution Cell Proliferation Assay (Promega, Wisconsin, USA). Based on a cell titration experiment to keep the control from reaching stationary phase at the time of analysis, about 10³ cells were plated to each well of a 96-well Falcon^{™} microplate and cultured overnight. The medium was then removed, and Drug 2 which is a derivative of a natural product combretastatin A-4 (anticancer agent; see structural formulae of Figures 9, 15, or 16) [CAS registration no.: 117048-59-6; as a reagent, e.g. Tokyo Chemical Industry Co., Ltd., product name Combretastatin A4 (product code: C2520)] or a precursor thereof (Prodrug 1; see structural formulae of Figures 9, 15, or 16: Katsunori Tanaka et al., NATURE COMMUNICATIONS, https://doi.org/10.1038/s41467-021-27804-5) was added to cancer cells (note that Drug 2 is an anticancer agent that inhibits polymerization of β-tubulin by binding to β-tubulin that configures intracellular microtubules with a disassociation constant at nanomolar (nM) levels to suppress proliferation of cancer cells, and Prodrug 1 is converted into Drug 2 by G41ArM-Ru) . 10 µL of compound in 10% DMSO/medium solution was added to 90 µL of medium to give a final DMSO concentration of 1%.

The GR50 value represents the concentration that gives half of maximum growth rate inhibitory rate. In order to evaluate the GR50 values of Prodrug 1 or Drug 2 against GSU cancer cells, various concentrations of Prodrug 1 (0, 0.01, 0.02, 0.04, 0.08, 0.1, 0.2, 0.4, 0.6, 1, 5, 25, 50, and 100 µM) or various concentrations of Drug 2 (0, 0.01, 0.02, 0.04, 0.08, 0.1, 0.2, 0.4, 0.6, and 1 µM) were tested (Figure 9). After 4 days of culture, the medium was exchanged to a culture medium (80 µL) with MTS reagent (20 µL). After incubating at 37°C for another 2 hours, endpoint absorbance was obtained at 490 nm via SpectraMax iD3 multi-mode microplate reader (Molecular Devices). The background control for this assay was a mixture of 20 µL of MTS reagent and 80 µL of medium without presence of cells. 100% growth was collected from cells incubated with 1% DMSO in the growth medium. The GR50 values obtained were calculated by GraphPad Prism (version 7.0d) software by employing fitting based on sigmoidal dose response formula.

For in cellulo prodrug activation research, G41ArM-Ru (10 µM) and various concentrations of Prodrug 1 (0.5, 1.0, 1.5, 2.0, 2.5, and 3.0 µM) were tested as control group (Figure 10). Drug 2 (1.0 µM) was used as positive control. The concentration of Prodrug 1 was changed (0.5, 1.0, 1.5, 2.0, 2.5, and 3.0 µM), and incubated together with a fixed concentration of G41ArM-Ru (10 µM). After 4 days incubation duration, the medium was exchanged to a solution of MTS reagent (20 µL) in growth medium (80 µL). After incubating at 37°C for another 2 hours, endpoint absorbance was obtained at 490 nm with SpectraMax iD3 multi-mode microplate reader (Molecular Devices). The background control for this assay was a mixture of 20 µL of MTS reagent and 80 µL of medium without presence of cells. 100% growth was obtained from cells incubated with 1% DMSO in the growth medium.

### Example 3

### Animal experiment

All animal experiments were carried out with approval from the animal ethics committee at RIKEN. In general, mice were anesthetized with oxygen containing 2.5% isoflurane at a flow rate of 2.5 - 3.0 L/min. GSU heterotransplanted tumor was established by subcutaneous injection of cells (about 1.0 x 10⁶ cells in 100 µl of non-nutrient RPMI1640) to the right shoulder of 6 weeks-old female nude mice BALB/cAJcl-nu/nu. Tumor growth was monitored while mice were accommodated in a facility with controlled temperature, salinity, aeration, and standard 12 hours light/dark cycle. Stock samples were prepared as follows. Prodrug 1 (19 mg) was first dissolved in DMSO (67 µl), then Tween 80 (142 µl) was added, and then 0.9% saline solution (1000 µl) was added. Drug 2 (3.9 mg) was first dissolved in DMSO (33 µl), then Tween 80 (60 µl) was added, and further 0.9% saline (510 µl) was added. G41ArM-Ru was prepared in PBS buffer (360 µl, 1 mM), and then 0.9% saline (840 µl) was added. On Day 1 post tumor transplantation, mice carrying GSU were randomized into 5 groups: vehicle control (Group 1, n = 4); Prodrug 1 only (Group 2, n = 4); Drug 2 only (Group 3, n = 4); G41ArM-Ru only (Group 4, n = 4); and G41ArM-Ru and Prodrug 1 (Group 5, n = 4). For each mouse, each injection volume never exceeded 100 µl. Following these guidelines, Group 1 received saline (100 µl); Group 2 received 33.5 mg/kg of Drug 2; Group 3 received 75.0 mg/kg of Prodrug 1; Group 4 received 300.0 mg/kg of G41ArM-Ru; and Group 5 received 300.0 mg/kg of G41ArM-Ru, and then 75.0 mg/kg of Prodrug 1. Treatment was performed every day for a total of 8 injections. Every day until Day 9 post injection, tumor volume and body weight of mice were recorded. Tumor volume was quantified with a caliper, and calculated as width 2 x length x 0.5. Mice were sacrificed on Day 9 post injection, and the tumor was resected, imaged, and weighed. In vivo metal catalyst reaction was performed in cancer model of mouse individuals, and the results of therapeutic effect by anti-cancer active material (Drug 2) are shown in Figure 11.

### Example 4

### 4. Tests based on patient-derived organoid (PDO)

### 4.1 Materials

In this research, the following pancreatic cancer patient-derived organoid (PDO) and the necessary medium were purchased from FUJIFILM Wako Pure Chemical Corporation (Tokyo, Japan). PDO was cultured under 5% CO₂ gas condition at 37°C. The specific media used were as follows.

**[Table 2]**

| Name | Type | Medium | KRAS mutant | Supplement |
|---|---|---|---|---|
| RPAN001 | Pancreatic cancer patient-derived organoid | Cancer cell proliferation medium + (032-25745) | G12D | 1 µM A-81-1 (039-24111) |
| | | | | 10 ng/ml bFGF (068-05384) |
| | | | | 100 ng/ml R-Spondin-1 (185-02804) |
| | | | | 10 µM Y-27632 (036-24023) |
| RPAN002 | Pancreatic cancer patient-derived organoid | Cancer cell proliferation medium + (032-25745) | Wild-type | 10 nM [leu15]-gastrin I (G9145) |
| | | | | 10 mM Nicotinamide (141-01202) |
| | | | | 100 ng/ml R-Spondin-1 (185-02804) |
| | | | | 10 µM Y-27632 (036-24023) |

### 4.2 Protocol for binding assay of sugar chain albumin 41 to PDO

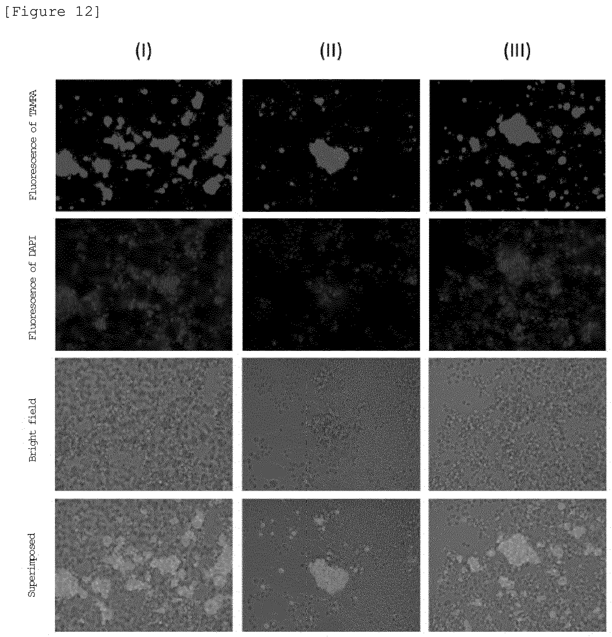

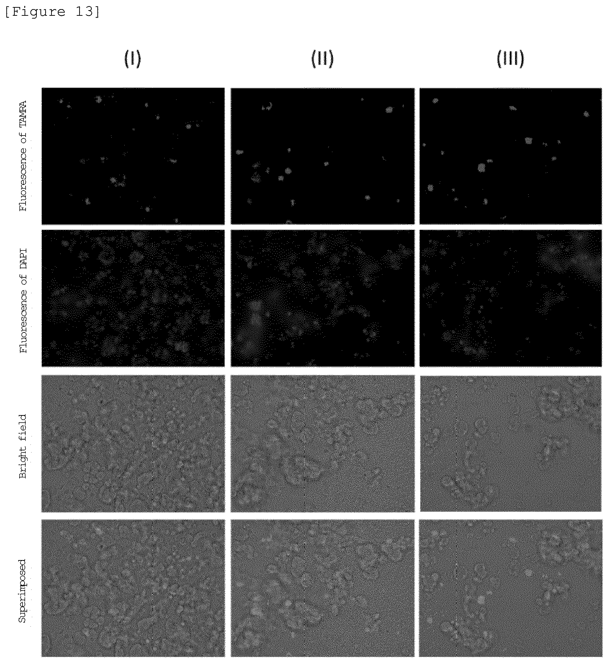

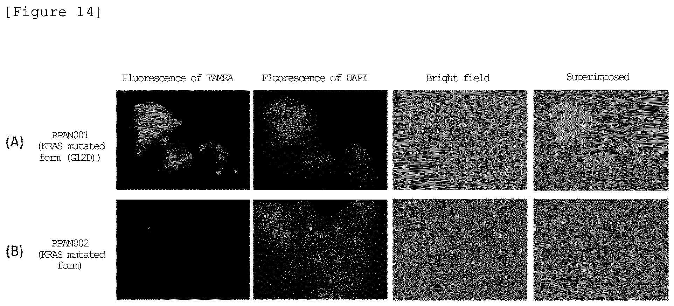

Pancreatic cancer PDO was transferred to a 96-well plate at a density of about 1 x 10⁴ per 150 µl of medium. Sugar chain albumin 41 stock solution was prepared at a concentration of 40 µM in the medium. In order to initiate incubation, 50 µl of sugar chain albumin 41 stock solution was added to 150 µl of PDO solution (final concentration was 10 µM of sugar chain albumin per well). PDO was then incubated at 37°C for 1 hour. PDO per well was then transferred to a 1.5 ml Eppendorf tube, centrifuged at 200 x g for 2 minutes, and washed 1 x with the medium (800 µl). Next, PDO was fixed by addition of 4% paraformaldehyde (100 µl), and incubated at room temperature for 5 minutes. The remaining medium and 4% paraformaldehyde were then removed by centrifugation at 200 x g for 2 minutes, and after 2 minutes of centrifugation at 200 x g, washed twice with PBS buffer (800 µl), and resuspended in PBS buffer (100 µl). Nucleus staining agent DAPI (10-fold dilution) was added to PDO, and this was incubated at room temperature for 5 minutes. Finally, PDO was transferred to a 96-well plate to prepared for microscopic observation with fluorescence microscope BZ-X710 (Keyence, Tokyo, Japan). Observation of TAMRA dye was measurement of fluorescence at λEX = 530 - 550 nm/λEM = 575 nm, and observation of DAPI at λEX = 330 - 385 nm/λEM = 460 nm. Images were observed and photographed at a magnification of 20x (Figure 12 - 13) and 40x (Figure 14).

### 4.3 Protocol of prodrug therapy by G41ArM-Ru against PDO

The survival rate of PDO was measured with ATP assay (oxidation of firely luciferin by D-luciferase catalyst is monitor with active cells in the presence of ATP and oxygen). The commercially available kit used in this research was CellTiter-Glo^{™} 2.0 Cell Viability Assay (Promega, Wisconsin, USA). PDO (RPAN001, about 1 x 10⁴ cells in the medium (150 µl) was plated to each well of a 96-well Falcon^{™} microplate, and then Drug 2 or Prodrug 1 was added to PDO. Ordinarily, 10 µL of compound in 10% DMSO/Media solution was added to 90 µL of medium to give a final DMSO concentration or 1%.

GR50 value indicates the concentration that renders half of maximum proliferation inhibitory rate. In order to evaluate the GR50 values of PDO (RPAN001), various concentrations of Prodrug 1 (0, 0.001, 0.005, 0.025, 0.0625, 0.125, 0.250, 0.5, 1, 2, 4, 8, and 16 µM) or various concentrations of Drug 2 (0, 0.001, 0.005, 0.025, 0.0625, 0.125, 0.250, 0.5, 1, 2, 4, 8, and 16 µM) were tested (Figure 15). After incubating for 4 days, ATP measurement reagent (100 µL) was added. After incubating at room temperature for another 10 minutes, endpoint luminescence was obtained at 578 nm with SpectraMax iD3 multi-mode microplate reader (Molecular Devices). The background control for this assay was a mixture of 100 µL of ATP assay reagent and 150 µL of medium in the absence of PDO. 100% proliferation was obtained from medium incubating PDO and 1% DMSO. The GR50 values obtained were calculated by GraphPad Prism (version 7.0d) software by employing fitting based on sigmoidal dose response formula.

For prodrug activation research, G41ArM-Ru at different concentrations (0.25, 0.5, 1, and 2.5 µM) was incubated together with a constant concentration of Prodrug 1 (1.0 µM) or without Prodrug 1. Drug 2 (1.0 µM) was used as positive control (Figure 16). After incubating for 4 days, ATP measurement reagent solution (100 µL) was added. After incubating at room temperature for another 10 minutes, endpoint luminescence was obtained at 578 nm with SpectraMax iD3 multi-mode microplate reader (Molecular Devices). The background control for this assay was a mixture of 100 µL of ATP assay reagent and 150 µL of medium in the absence of PDO. 100% growth was PDO incubated with 1% DMSO in the growth medium.

### Example 5

### 5. Target selectivity of glycoalbumin 41 in patient tumor tissue transplantation (Patient-derived xenografts, PDX) model

To heterotransplanted PDX mice of pancreatic cancer patient-derived organoid (PDO) (RPAN001, mutated KRAS gene (G12D)) or (B) pancreatic cancer patient-derived organoid (PDO) (RPAN002, wild-type KRAS gene), 100 µL of sugar chain albumin 41 labeled with 50 µM of Hilyte Fluor 750 was intravenously injected. This was photographed 2 hours after injection with Clair Vivo from SHIMADZU. Mice were irradiated at the same photographing time and strength. The fluorescence excitation wavelength was adjusted to 785 nm and emission wavelength to 849 nm, and 5 seconds of exposure was carried out.

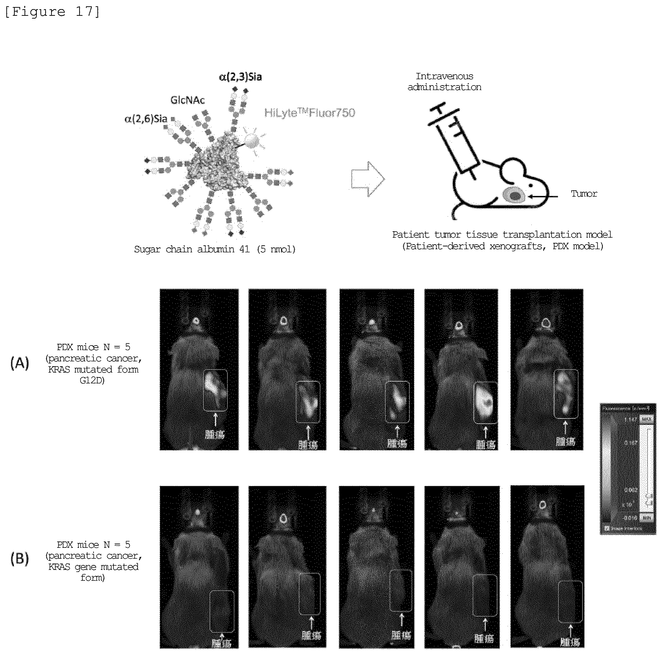

Figure 17 shows in vivo imaging photographed as above. As is clear from Figure 17, it was shown that sugar chain albumin 41 selectively targets cancer carrying KRAS gene mutation (G12D).

### Example 6

### 6. In vivo prodrug activation by G41ArM-Ru against proliferation of pancreatic tumor having KRAS gene mutation (G12D) in PDX mice

All animal experiments were carried out with approval from the animal ethics committee at RIKEN. Mice were anesthetized with oxygen containing 2.5% isoflurane at a flow rate of 2.5 - 3.0 L/min. Pancreatic cancer patient-derived organoid (PDO) (RPAN001, mutated KRAS gene (G12D)) heterotransplanted tumor was established by subcutaneous injection of PDO to the right shoulder of 6 weeks-old female mice NOD.Cg-Prkdcscld//2rgtm1wjl/szj. Tumor growth was monitored while mice were accommodated in a facility with controlled temperature, salinity, aeration, and standard 12 hours light/dark cycle.

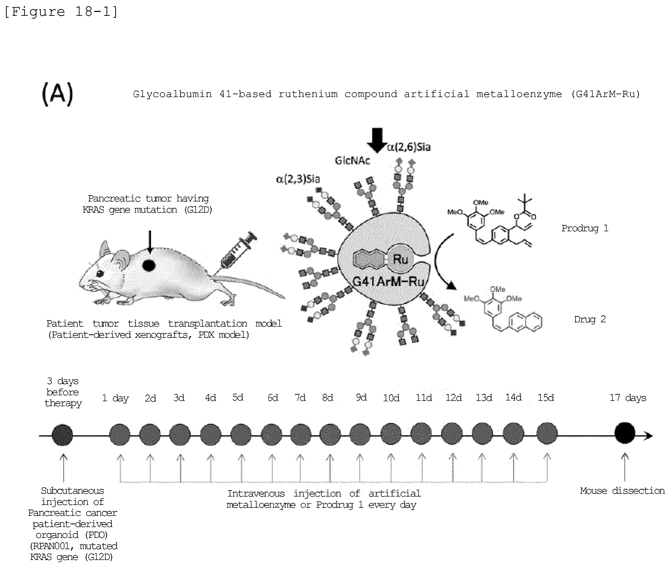

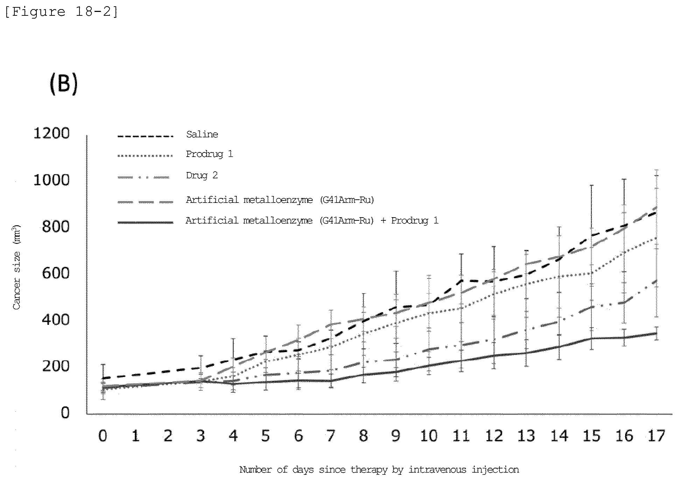

Stock samples were prepared as follows. In other words, Prodrug 1 (19 mg) was first dissolved in DMSO (67 µl), then Tween 80 (142 µl) was added, and then 0.9% saline solution (1000 µl) was added. Drug 2 (3.9 mg) was first dissolved in DMSO (33 µl), then Tween 80 (60 µl) was added, and further 0.9% saline (510 µl) was added. G41ArM-Ru was prepared in PBS buffer (360 µl, 1 mM), and then 0.9% saline (840 µl) was added. On Day 1 post tumor transplantation, mice carrying GSU were randomized into 5 groups: vehicle control (Group 1, n = 4); Prodrug 1 only (Group 2, n = 4); Drug 2 only (Group 3, n = 4); G41ArM-Ru only (Group 4, n = 4); and G41ArM-Ru and Prodrug 1 (Group 5, n = 4). For each mouse, each injection volume never exceeded 100 µl. Following these guidelines, Group 1 received saline (100 µl); Group 2 received 45.8 mg/kg of Drug 2; Group 3 received 87.5 mg/kg of Prodrug 1; Group 4 received 225.0 mg/kg of G41ArM-Ru; and Group 5 received 225.0 mg/kg of G41ArM-Ru, and then 87.5 mg/kg of Prodrug 1. Following the treatment plan of prodrug therapy (Figure 18A), Treatment was performed every day for a total of 15 injections. Every day until Day 17 post injection, tumor volume and body weight of mice were recorded. Tumor volume was quantified with a caliper, and calculated as width 2 x length x 0.5. Mice were sacrificed on Day 17 post injection, and the tumor was resected, imaged, and weighed. In vivo metal catalyst reaction was performed in cancer model of mouse individuals, and the results of therapeutic effect by anti-cancer active material (Drug 2) are shown in Figure 18B.

The results of tumor weight on Day 17 were saline (n = 4), Drug 2 (45.8 mg/kg, n = 4), Prodrug 1 (87.5 mg/kg, n = 4), G41ArM-Ru (225.0 mg/kg, n = 4), and co-treatment of G41ArM-Ru and Prodrug 1 (225.0 mg/kg and 87.5 mg/kg, n = 4), and it was shown that G41ArM-Ru and Prodrug 1 exert a synergistic effect at target tumor site.

## Claims

1. A production method of a protein that selectively recognizes K-ras gene mutation cancer, comprising:
a first step of conjugating the following sugar chain to lysine residues on the protein molecule; 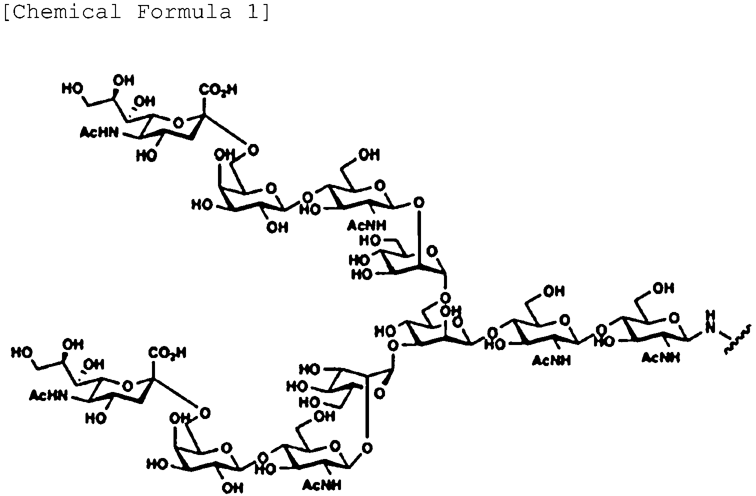
a second step of conjugating the following sugar chain to lysine residues on said molecule; and 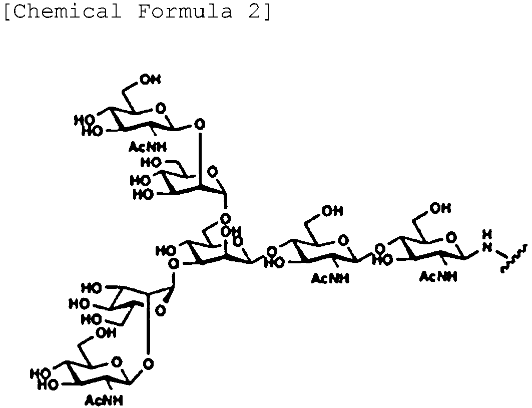
a third step of conjugating any of multiantennary complex sugar chains to lysine residues on said molecule.

2. The production method according to claim 1, wherein said multiantennary complex sugar chain is at least one type of biantennary complex sugar chain.

3. The production method according to claim 2, wherein in the third step, at least one of sugar chain selected from the group consisting of the following is conjugated: 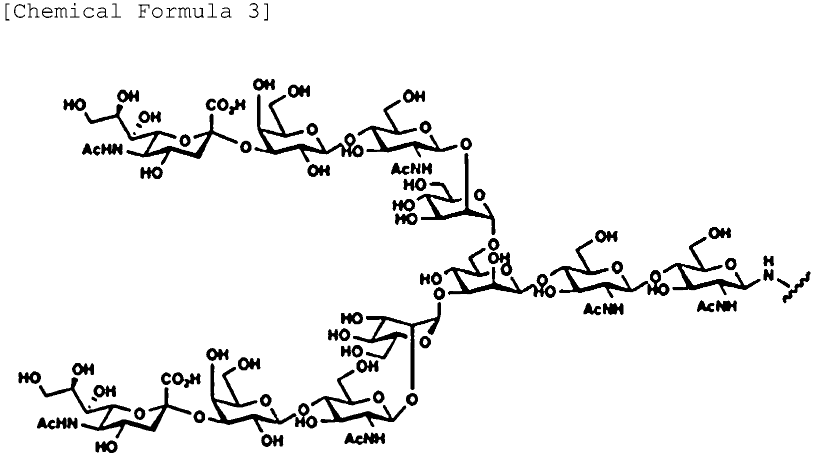 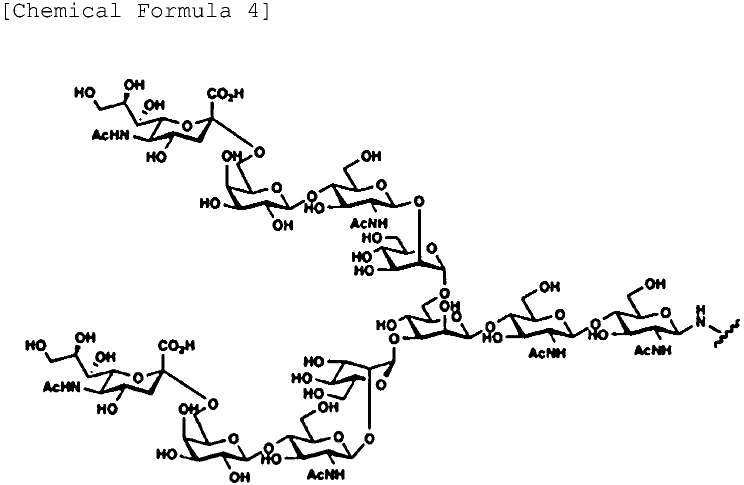 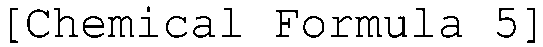 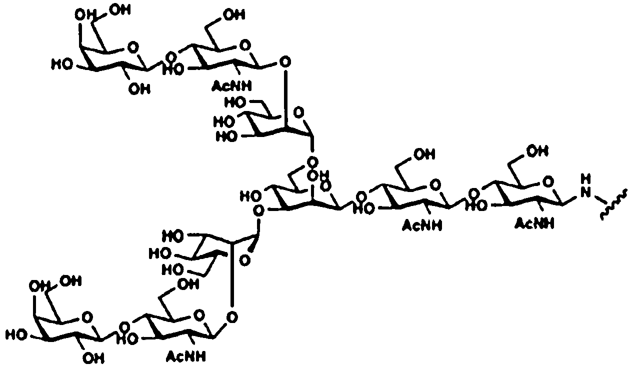 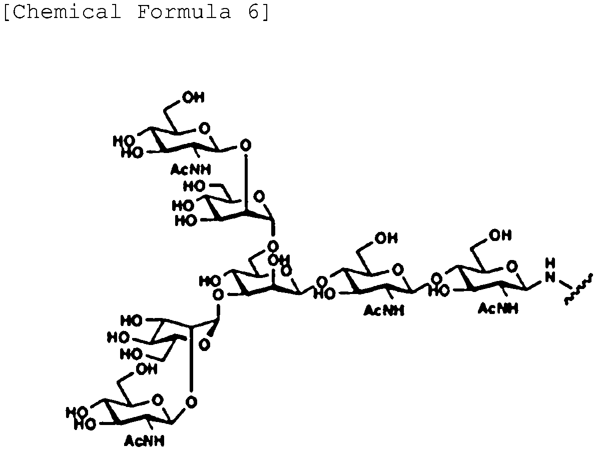 and 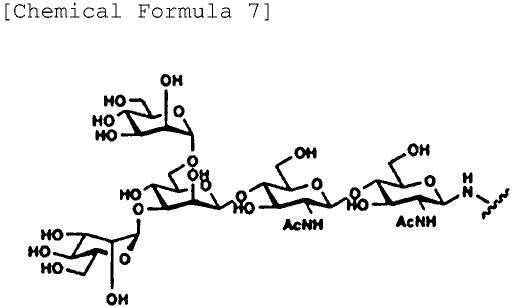 .

4. The production method according to claim 2, wherein in the third step, at least any one of the following sugar chains is conjugated: 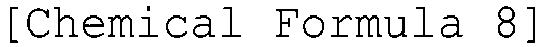 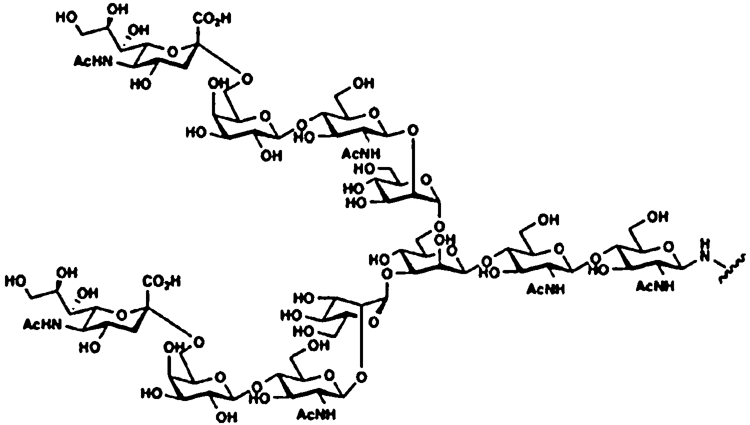 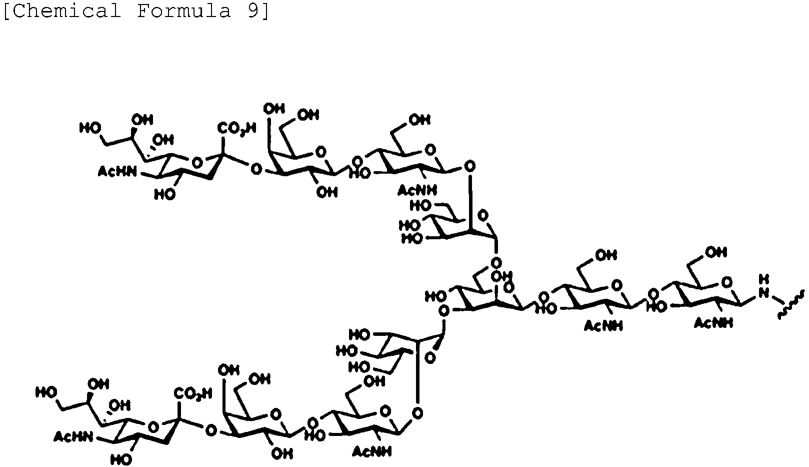 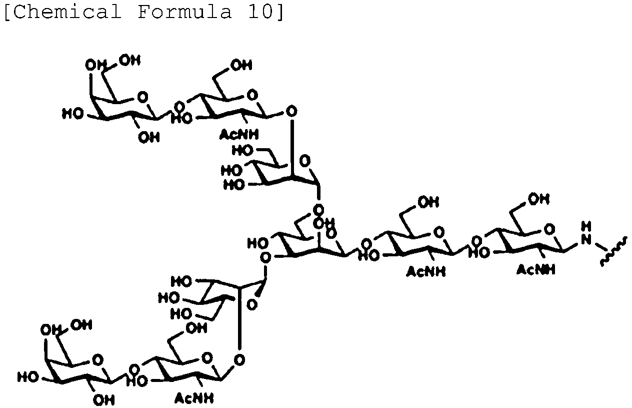 ; and 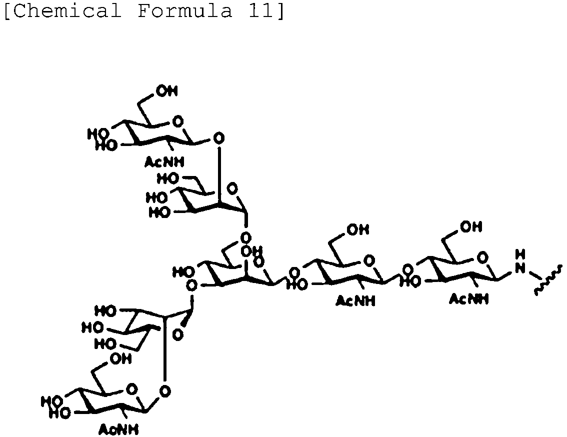 .

5. The production method according to claim 1, wherein said protein is albumin.

6. The method according to claim 1, wherein in each of said first to third steps, 2 to 5 sugar chains are respectively conjugated.

7. The method according to claim 1, wherein in each of said first to third steps, about 3 sugar chains are respectively conjugated.

8. The production method according to claim 1, wherein said "protein that selectively recognizes K-ras gene mutation cancer" comprises 9 to 11 sugar chains.

9. The production method according to claim 1, wherein said "K-ras gene mutation" comprises a G12D mutation.

10. The production method according to claim 1, wherein said "K-ras gene mutation cancer" is gastric adenocarcinoma or pancreatic cancer.

11. The production method according to claim 5, wherein said albumin accommodates a metal catalyst in its hydrophobic spot so that it is not exposed or not substantially exposed to the hydrophilic environment.

12. The production method according to claim 11, wherein said hydrophobic spot is albumin drug binding site I (drug site I).

13. The production method according to claim 11, wherein said metal catalyst is bound to said hydrophobic spot via a ligand against said albumin.

14. The production method according to claim 13, wherein said ligand is selected from the group consisting of warfarin, azapropazone, acenocoumarol, phenylbutazone, salicylate salt, indomethacin, phenytoin, tolbutamide, chlorpropamide, iophenoxate, iodipamide, sulfadimethoxine, phenprocoumon, glibenclamide, sulfathiazole, tenoxicam, camptothecin, prodan, bilirubin, eicosanoid, and carboxy-methyl-propyl-furan propanoate (uremic toxin), and coumarin.

15. The production method according to claim 13, wherein said metal catalyst is bound to said hydrophobic spot via a linker bound to said ligand.

16. The production method according to claim 15, wherein said linker is an alkyl chain or a polyethylene glycol (PEG) chain having amino and carboxyl groups on both ends.

17. The production method according to claim 16, wherein said linker is C₁ - C₃ alkyl.

18. The production method according to claim 11, wherein said metal catalyst is a ruthenium catalyst.

19. A protein that selectively recognizes K-ras gene mutation cancer that can be produced by the production method according to any one of claims 1 to 18.

20. A pharmaceutical composition for cancer therapy comprising the protein according to claim 19.
